(19) Europäisches Patentamt
European Patent Office
Office européen des brevets

(11) EP 0 962 457 B1

(12) EUROPEAN PATENT SPECIFICATION

(45) Date of publication and mention of the grant of the patent:
**06.08.2003 Bulletin 2003/32**

(51) Int Cl.[7]: **C07D 401/14**, A61K 31/445, C07D 401/04, C07D 405/14, C07D 405/04

(21) Application number: **99304238.1**

(22) Date of filing: **01.06.1999**

(54) **Piperidones as tachykinin antagonists**

Piperidone als Tachykinin-Hemmer

Piperidones comme antagonistes de la tachykinine

(84) Designated Contracting States:
**AT BE CH CY DE DK ES FI FR GB GR IE IT LI LU NL PT SE**

(30) Priority: **04.06.1998 GB 9812037**

(43) Date of publication of application:
**08.12.1999 Bulletin 1999/49**

(73) Proprietors:
- **Pfizer Limited**
  **Sandwich, Kent CT13 9NJ (GB)**
  Designated Contracting States:
  **GB**
- **PFIZER INC.**
  **New York, N.Y. 10017 (US)**
  Designated Contracting States:
  **BE CH DE DK ES FI FR GR IE IT LI LU NL SE AT CY**

(72) Inventors:
- **Fox, David Nathan Abraham**
  **Sandwich, Kent CT13 9NJ (GB)**
- **Mathias, John Paul**
  **Sandwich, Kent CT13 9NJ (GB)**
- **Tommasini, Ivan**
  **Sandwich, Kent CT13 9NJ (GB)**

(74) Representative: **Ruddock, Keith Stephen et al**
**Pfizer Limited,**
**European Patent Department,**
**Ramsgate Road**
**Sandwich, Kent CT13 9NJ (GB)**

(56) References cited:
**EP-A- 0 790 248**
**EP-A- 0 791 592**
**WO-A-96/05193**
**WO-A-97/19942**
**WO-A-97/27185**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 962 457 B1

## Description

This invention relates to piperidones. More particularly, this invention relates to 5-(2-[azetidin-1-yl)ethyl])piperidin-2-one derivatives and to processes for the preparation of, intermediates used in the preparation of, compositions containing and uses of, such derivatives.

The present compounds are antagonists of tachykinins, including neurokinin A (NKA), neurokinin B (NKB) and Substance P, acting at the human neurokinin-1 ($NK_1$), neurokinin-2 ($NK_2$) or neurokinin-3 ($NK_3$) receptor, or any combination thereof. The derivatives are therefore useful for treating an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, a disease caused by Helicobacter pylori or other urease-positive Gram negative bacteria, an urogenital tract disorder such as incontinence, impotence, hyperreflexia or cystitis, a pulmonary disorder such as chronic obstructive airways disease, an allergy such as eczema, contact dermatitis or rhinitis, a hypersensitivity disorder such as to poison ivy, a vasospastic disease such as angina or Reynaud's disease, a fibrosing or collagen disease such as scleroderma or eosinophillic fascioliasis, reflux sympathetic dystrophy such as shoulder/hand syndrome, an addiction disorder such as alcoholism, a stress-related somatic disorder, a peripheral neuropathy such as diabetic neuropathy, neuralgia, causalgia, painful neuropathy, a burn, herpetic neuralgia or post-herpetic neuralgia, a neuropathological disorder such as Alzheimer's disease or multiple sclerosis, a disorder related to immune enhancement or suppression such as systemic lupus erythematosis, a rheumatic disease such as fibrositis, emesis, cough, acute or chronic pain, migraine, or an ophthalmic disease such as proliferative retinopathy.

WO 97/19942 discloses 5-azabicyclo (3.1.0) hexylalkyl-2-piperidones and -glutarimides as NK receptor antagonists. EP 0791592 discloses azetidines that find utility as tachykinin antagonists. EP 0790248 discloses 3-aza-piperidone-(tetrahydropyrimidin-2-one) and 3-oxa-piperidone (1,3 oxazin-2-one) derivatives which are useful as tachykinin/neurokinin antagonists.

WO-A-96/05193 and WO-A-97/27185 disclose azetidinylalkyllactam derivatives with tachykinin antagonist activity. With respect to this prior art, the present compounds have been surprisingly found to be more potent $NK_2$ receptor antagonists and/or to have increased metabolic stability and/or to have improved selectivity as antagonists for the $NK_2$ receptor as opposed to the $NK_3$ receptor.

As stated above, the present compounds are particularly potent and selective antagonists of tachykinins, including NKA, NKB and Substance P, acting at the human $NK_2$ receptor. They are therefore particularly useful for treating an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain.

The present invention provides a compound of the formula:-

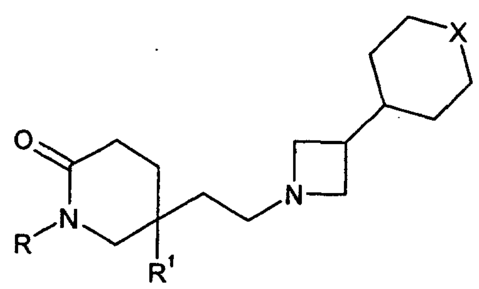

**(I)**

or a pharmaceutically acceptable acid addition salt or solvate thereof, wherein

R is $C_3$-$C_7$ cycloalkyl, ($C_3$-$C_7$ cycloalkyl)$C_1$-$C_4$ alkylene or benzyl;

$R^1$ is phenyl optionally substituted by 1 or 2 substituents each independently selected from fluoro and chloro;
X is O or $NSO_2R^2$; and
$R^2$ is $C_1$-$C_4$ alkyl or halo($C_1$-$C_4$)alkyl.

In the above definition of a compound of the formula (I), an alkyl group containing 3 or 4 carbon atoms and an alkylene group containing 2 or more carbon atoms may be straight- or branched-chain.
Preferred $C_3$-$C_7$ cycloalkyl groups are cyclopropyl, cyclobutyl, cyclopentyl and cyclohexyl.
Preferred $C_1$-$C_4$ alkylene groups are methylene and 1,2-ethylene.
Preferred, $C_1$-$C_4$ alkyl groups are methyl and ethyl.
Preferably, R is ($C_3$-$C_7$ cycloalkyl)$C_1$-$C_4$ alkylene.
Preferably, R is cyclopentyl, cyclohexyl, cyclopropylmethyl, 2-cyclopropylethyl, cyclohexylmethyl or benzyl.
Preferably, R is cyclopropylmethyl.
Preferably, $R^1$ is 3,4-difluorophenyl, 3,4-dichlorophenyl or 4-chloro-3-fluorophenyl.
Preferably, $R^1$ is 3,4-dichlorophenyl.
Preferably, X is $NSO_2$($C_1$-$C_4$ alkyl).
Preferably, X is O, $NSO_2CH_3$ or $NSO_2CH_2CH_3$.
Preferably, X is $NSO_2CH_3$.
Preferably, $R^2$ is $C_1$-$C_4$ alkyl.
Suitable acid addition salts are formed from acids which form non-toxic salts and examples include the hydrochloride, hydrobromide, hydroiodide, sulphate, hydrogen sulphate, nitrate, phosphate, hydrogen phosphate, acetate, maleate, fumarate, lactate, tartrate, citrate, gluconate, succinate, benzoate, methanesulphonate, benzenesulphonate, p-toluenesulphonate, 5-sulphosalicylate and 10-camphorsulphonate salts.
For a review on suitable acid addition salts that may be used see Berge et al, J. Pharm. Sci., 66, 1-19 (1977).
The pharmaceutically acceptable solvates of the compounds of the formula (I) include the hydrates thereof.
Also included within the present scope of the compounds of the formula (I) are polymorphs and radiolabelled derivatives thereof.
A compound of the formula (I) contains at least one asymmetric carbon atom and therefore exists in two or more stereoisomeric forms. The present invention includes the individual stereoisomers of the compounds of the formula (I) and mixtures thereof.
Separation of diastereoisomers may be achieved by conventional techniques, e.g. by fractional crystallisation, chromatography or H.P.L.C. of a stereoisomeric mixture of a compound of the formula (I) or a suitable salt or derivative thereof. An individual enantiomer of a compound of the formula (I) may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid.
The preferred compounds of the formula (I) have the (S)-stereochemistry at the 5-position of the piperidin-2-one ring, i.e.

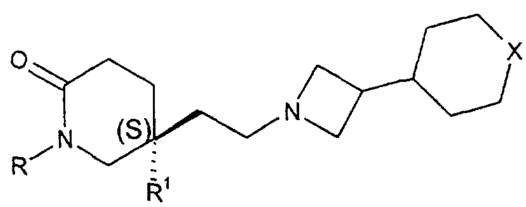

(IA)

wherein R, $R^1$ and X are as previously defined for a compound of the formula (I).
Preferred compounds of the formula (I) are those of the Examples hereafter.
A preferred compound of the formula (I) is 5(S)-1-(cyclopropylmethyl)-5-(3,4-dichlorophenyl)-5-(2-[3-(1-[methylsulfonyl]-4-piperidinyl)-1-azetanyl]ethyl)tetrahydro-2(1H)-pyridinone or a pharmaceutically acceptable acid addition salt or solvate thereof.
The compounds of the formula (I) provided by the invention can be prepared by the following methods wherein R, $R^1$, $R^2$ and X are as previously defined for a compound of the formula (I) unless otherwise stated.
1) The compounds of the formula (I) can be prepared by reductive amination using as starting materials a

compound of the formula:-

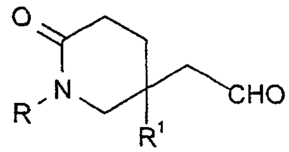

(II)

and a compound of the formula:-

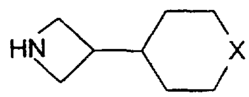

(III)

, or an acid addition salt thereof. The reaction is preferably carried out in the presence of a suitable acid, e.g. acetic acid.

The reaction proceeds via the initial formation of an intermediate iminium salt of the formula:-

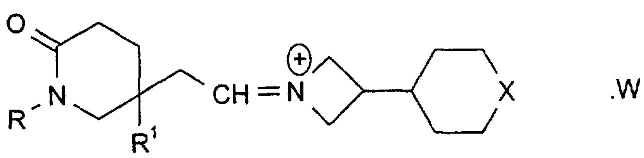

(IV)

where W is an anion (e.g. acetate) derived from a suitable acid (e.g. acetic acid), which may be stable and isolatable. The reaction is preferably carried out without isolation of the intermediate of the formula (IV) in which case it is reduced in situ to provide a compound of formula (I).

In a typical procedure, an aldehyde of the formula (II) is mixed with an azetidine of the formula (III) in a suitable solvent, e.g. tetrahydrofuran or dichloromethane, and the mixture is then treated with a suitable reducing agent, e.g. sodium triacetoxyborohydride or sodium cyanoborohydride, in the presence of a suitable acid, e.g. acetic acid, to give the required product. If an acid addition salt of an azetidine of the formula (III) is used as a starting material, a suitable acid acceptor, e.g. triethylamine, is preferably added prior to the addition of the reducing agent and no additional acid is necessary.

The reaction is typically carried out at room temperature.

The compounds of the formula (III) can be prepared by conventional procedures.

The aldehydes of the formula (II) can be prepared by conventional methods such as by those described in WO-A-96/05193 and WO-A-97/27185, or by analogous preparations thereto.

2) The compounds of the formula (I) can be prepared by alkylation of a N-deprotonated form of a compound of the formula:

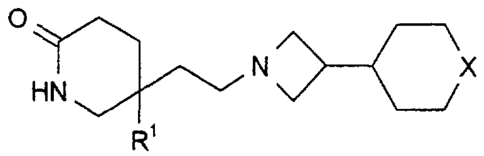

(V)

with a compound of the formula:

$$R\text{-}Y^1 \quad (VI)$$

wherein $Y^1$ is a suitable leaving group, e.g. halo, preferably chloro, bromo or iodo, methanesulphonyloxy or para-toluenesulphonyloxy.

In a typical procedure, a compound of the formula (V) is first deprotonated with a suitable base, e.g. sodium hydride, and then alkylated in situ with a compound of the formula (VI) where $Y^1$ is preferably bromo or methanesulphonyloxy. The reaction is typically carried out in a suitable solvent, e.g. dimethylformamide.

Alternatively, the rection can be carried out by reacting the starting materials of the formulae (V) and (VI) together in the presence of a suitable base, e.g. potassium hydroxide, and in a suitable solvent, e.g. dimethylsulphoxide, at about room temperature. If a compound of the formula (VI) where $Y^1$ is chloro is used, potassium iodide may also be added to increase the rate of reaction.

The starting materials of the formula (V) can be prepared by conventional methods such as by similar preparations to those described in WO-A-96/05193 and WO-A-97/27185.

The starting compounds of the formula (VI) can be prepared by conventional methods.

3) The compounds of the formula (I) can be prepared by reaction of a compound of the formula:

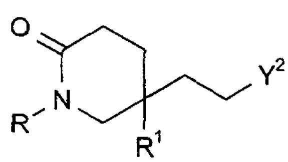

(VII)

wherein $Y^2$ is a suitable leaving group, e.g. chloro, bromo, iodo, methanesulphonyloxy, trifluoromethanesulphonyloxy or p-toluenesulphonyloxy, with a compound of the formula (III). The compound of the formula (III) may be generated in situ from an acid addition salt thereof using a suitable acid acceptor.

In a typical procedure, a compound of the formula (VII) is reacted with a compound of the formula (III), or an acid addition salt thereof, in the presence of a suitable acid acceptor, e.g. triethylamine or potassium carbonate, and in a suitable solvent, e.g. acetonitrile.

The starting materials of the formula (VII) can be prepared by conventional methods such as by those described in WO-A-96/05193 and WO-A-97/27185 or by analogous preparations thereto.

All of the above reactions and the preparations of novel starting materials used in the preceding methods are conventional and appropriate reagents and reaction conditions for their performance or preparation as well as procedures for isolating the desired products will be well known to those skilled in the art with reference to literature precedents and the Examples and Preparations hereto.

A pharmaceutically acceptable acid addition salt of a compound of the formula (I) may be readily prepared by mixing together solutions of a compound of the formula (I) and the desired acid. The salt may precipitate from solution and be collected by filtration or may be recovered by evaporation of the solvent.

The affinity of the compounds of formula (I) and their salts for the human $NK_1$ receptor can be tested in vitro by testing their ability to inhibit [$^3$H]-Substance P binding to membranes prepared from the human IM9 cell line expressing the human $NK_1$ receptor using a modification of the method described in McLean, S. et al, J. Pharm. Exp. Ther., 267, 472-9 (1993) in which whole cells were used.

The affinity of the compounds of formula (I) and their salts for the human $NK_2$ receptor can be tested in vitro by testing their ability to compete with [$^3$H]-NKA (neurokinin A) for binding to membranes prepared from Chinese hamster ovary cells expressing the cloned human $NK_2$ receptor. In this method, washed Chinese hamster ovary cell membranes are prepared as described for the previous method where IM9 cells are used instead. The membranes are incubated (90 min, 25°C) with [$^3$H]-NKA and with a range of concentrations of the test compound. Non-specific binding was determined in the presence of 10μM NKA.

The $NK_2$ receptor antagonist activity of the compounds of the formula (I) can be tested, in vitro, by testing their ability to antagonise the contractile effects of the selective $NK_2$ receptor agonist [βAla$^8$]$NKA_{(4-10)}$ in the rabbit pulmonary artery, using the method of Patacchini and Maggi, Eur. J. Pharmacol., 236, 31-37 (1993).

The compounds of the formula (I) and their salts can be tested for $NK_2$ receptor antagonist activity, in vivo, by testing their ability to inhibit bronchoconstriction induced by [βAla$^8$]$NKA_{(4-10)}$ in the anaesthetised guinea pig, using the method described by Murai et al, J. Pharm. Exp. Ther., 262, 403-408 (1992) or Metcalfe et al, Br. J. Pharmacol., 112, 563P (1994).

The compounds of the formula (I) and their salts can be tested for $NK_3$ receptor affinity, in vitro, by testing their ability to inhibit [$^3$H]-senktide binding to membranes from guinea pig cortex using the method of Guard, et al, Br. J.Pharmacol., 99, 767-773 (1990).

The in vitro metabolic stability of the compounds of the formula (I) and their salts can be tested by incubating samples for 1 hour at 37°C in the presence of a "moderate activity" (re. concentrations of cytochrome p450 enzymes present) human liver microsomal preparation (prepared from human liver homogenates). The samples were analysed by HPLC at regular intervals during this one hour period and the half-life (t½) determined (in minutes).

For human use, the compounds of the formula (I) and their salts can be administered alone, but will generally be administered in admixture with a pharmaceutically acceptable diluent or carrier selected with regard to the intended route of administration and standard pharmaceutical practice. For example, they can be administered orally, including sublingually and buccally, in the form of tablets containing such excipients as starch or lactose, or in capsules or ovules either alone or in admixture with excipients, or in the form of elixirs, solutions or suspensions containing flavouring or colouring agents. They can be injected parenterally, for example, intravenously, intramuscularly or subcutaneously. For parenteral administration, they are best used in the form of a sterile aqueous solution which may contain other substances, for example, enough salts or glucose to make the solution isotonic with blood.

For oral and parenteral administration to human patients, the daily dosage level of the compounds of the formula (I) and their salts will be from 0.001 to 20, preferably from 0.01 to 20, and most preferably from 0.1 to 10, mg/kg (in single or divided doses). Thus tablets or capsules of the compounds will contain from 0.1 to 500, preferably from 10 to 200, mg of active compound for administration singly or two or more at a time, as appropriate. The physician in any event will determine the actual dosage which will be most suitable for an individual patient and it will vary with the age, weight and response of the particular patient. The above dosages are exemplary of the average case; there can, of course, be individual instances where higher or lower dosage ranges are merited, and such are within the scope of this invention.

Alternatively, the compounds of the formula (I) can be administered intranasally, by inhalation or in the form of a suppository or pessary, or they may be applied topically in the form of a lotion, solution, cream, ointment or dusting powder. An alternative means of transdermal administration is by use of a skin patch. For example, they can be incorporated into a cream comprising an aqueous emulsion of polyethylene glycols or liquid paraffin, or they can be incorporated, at a concentration of between 1 and 10% by weight, into an ointment consisting of a white wax or white soft paraffin base together with such stabilisers and preservatives as may be required. The compounds of the formula (I) may also be administered in the form of an aerosol spray presentation or by using a dry powder inhaler formulation.

It is to be appreciated that reference to treatment includes curative, palliative and prophylactic treatment.

Thus the invention provides:-

(i) a compound of the formula (I) or a pharmaceutically acceptable acid addition salt or solvate thereof;
(ii) processes for the preparation of a compound of the formula (I) or a pharmaceutically acceptable acid addition salt or solvate thereof;
(iii) a pharmaceutical composition comprising a compound of the formula (I), or a pharmaceutically acceptable acid addition salt or solvate thereof, together with a pharmaceutically acceptable diluent or carrier;
(iv) a compound of the formula (I), or a pharmaceutically acceptable acid addition salt, solvate or composition thereof, for use as a medicament;
(v) the use of a compound of the formula (I), or of a pharmaceutically acceptable acid addition salt, solvate or composition thereof, for the manufacture of a medicament for the treatment of a disease by producing an antagonist effect on a tachykinin acting at the human $NK_1$, $NK_2$ or $NK_3$ receptor, or any combination thereof;
(vi) use as in (v) where the disease is an inflammatory disease such as arthritis, psoriasis, asthma or inflammatory bowel disease, a central nervous system (CNS) disorder such as anxiety, depression, dementia or psychosis, a

gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropoathy such as diabetic neuropathy, neuralgia, causalgia, painful neuropathy, a burn, herpetic neuralgia or post-herpetic neuralgia, cough or acute or chronic pain;
(vii) a compound of the formula (III), or an acid addition salt thereof;
(viii) a compound of the formula (IV) and
(ix) a compound of the formula (V).

In the following experimental section,

"Ac" means acetyl,
"Me" means methyl,
"Et" means ethyl,
"Ph" means phenyl,
"Ms" means mesyl,
"Ts" means tosyl,
"THF" means tetrahydrofuran,
and "DCM" means dichloromethane.

It should also be noted that the azetane nomenclature used in the experimental section is the IUPAC system equivalent of azetidine.
The following Examples illustrate the preparation of the compounds of the formula (I):

EXAMPLE 1

(5S)-*1*-(Cyclopropylmethyl)-5-(3,4-dichlorophenyl)-5-(2-[3-[1-(methylsulfonyl)-4-piperidinyl]-1-azetanyl]ethyl) tetrahydro-2(1*H*)-pyridinone

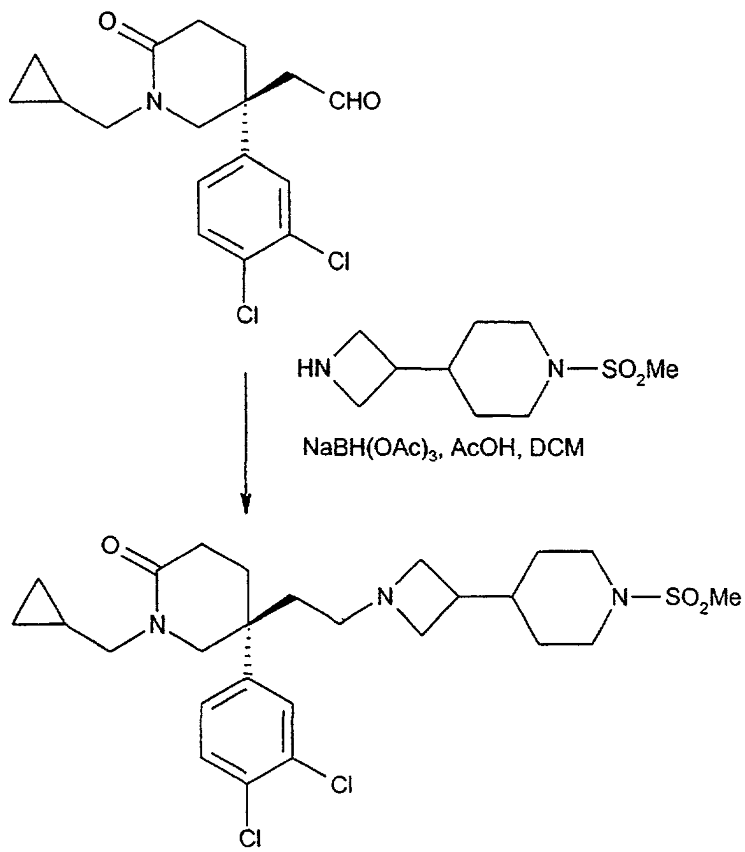


To a solution of the aldehyde (3.20g, 9.40mmol) (see WO-A-96/05193, Example 123) and 4-(3-azetanyl)-1-(methylsulfonyl)piperidine (2.05g, 9.40mmol) (see Preparation 1) in dry dichloromethane (70ml) under nitrogen was added glacial acetic acid (0.65ml, 11.28mmol), followed immediately by sodium triacetoxyborohydride (2.80g, 13.17mmol). After stirring for 3.5 hours, the reaction was quenched with ca. 5ml of glacial acetic acid and stirred for 20 minutes. After this time the resultant mixture was diluted with dichloromethane (50ml) and partitioned between, firstly, water and dichloromethane, then 2M aqueous sodium hydroxide solution and dichloromethane, the organic portion being retained between the aqueous washings. Finally, the organic portion was dried over sodium sulphate. The mixture was filtered and the solvent removed by evaporation under reduced pressure to produce a white foam. This foam was dissolved in a minimum quantity of dichloromethane and purified by column chromatography on silica gel eluting with a solvent gradient of dichloromethane:methanol:ammonia (100:0:0, by volume) changing to dichloromethane:methanol:ammonia (94:5:1, by volume), to provide the title compound (3.38g). TLC $R_f$ = 0.2 (silica, methanol:dichloromethane, 1:19, by volume). LRMS m/z = 542.2 $(m+1)^+$.
Found: C,56.52; H,7.01; N,7.43. $C_{26}H_{37}Cl_2N_3SO_3$. 0.5 $H_2O$ requires C,56.61; H,6.93; N,7.61%.
$^1$H-NMR ($CDCl_3$): $\delta$ = 0.2-0.4 (m,2H), 0.5-0.7 (m,2H), 1-1.3 (m,4H), 1.4-1.9 (m,4H), 1.9-2.2 (m, 6H), 2.2-3 (m, 8H), 3-3.6 (5H,m), 3.7-3.9 (m,3H), 7.1-7.2 (m,1H), 7.3-7.5 (m,2H).

EXAMPLES 2 to 7

The compounds of the following tabulated Examples of the general formula:

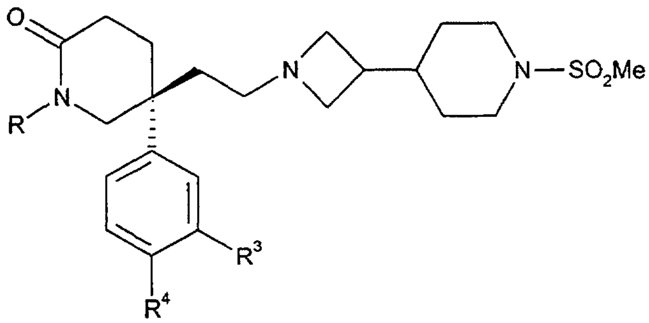

were prepared by a similar method to that of Example 1 using the appropriate aldehyde starting materials and the azetane of Preparation 1.

| Example No. | R | $R^3$ | $R^4$ | LRMS m/z = | Analytical data |
|---|---|---|---|---|---|
| 2[1] | 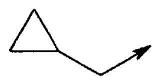 | F | Cl | 526.4 $(m+1)^+$ | $^1$H-NMR ($CDCl_3$): δ = 0.2-0.4 (m,2H), 0.5-0.7 (m,2H), 1-1.1 (m,1H), 1.1-1.5 (m,4H), 1.5-1.9 (m,6H), 1.9-2.3 (m,6H), 2.3-2.4 (m,1H), 2.5-2.7 (m,4H), 3.1-3.5 (m,5H), 3.7-3.9 (m,3H), 7-7.2 (m,2H), 7.3-7.4 (m,1H).<br>Found: C,56.52; H,7.02; N,7.43. $C_{26}H_{37}Cl_2N_3O_3S$. 0.5 $H_2O$ requires C,56.61; H,6.93; N,7.61%. |
| 3[2] | 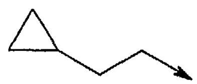 | Cl | Cl | 556.6 $(m+1)^+$ | $^1$H-NMR ($CDCl_3$): δ = 0.1-0.2 (m,2H). 0.4-0.6 (m,2H), 0.6-0.8 (m,1H), 1.1-1.9 (m,9H), 1.9-2.8 (m,14H), 3.1-3.5 (m,4H), 3.5-3.7 (m,2H), 3.7-3.9 (m,2H), 7-7.2 (m,1H), 7.3-7.5 (m,2H). |
| 4[3] | 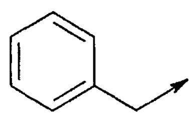 | Cl | Cl | 580.1 $(m+1)^+$ | $^1$H-NMR ($CDCl_3$): δ = 1-1.3 (m,2H), 1.3-1.8 (m,5H), 1.8-2.3 (m,6H), 2.3-2.9 (m,8H), 3.1-3.3 (m,3H), 3.3-3.9 (m,3H), 4.3-4.5 (m,1H), 4.7-4.9 (m,1H), 6.8-6.9 (m,1H), 7-7.4 (m,7H).<br>Found: C,57.47; H,6.44; N,6.69. $C_{29}H_{37}Cl_2N_3O_3S$. 1.5 $H_2O$ requires C,57.51; H,6.66; N,6.94%. |
| 5[4] | 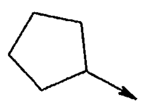 | Cl | Cl | 556.4 $(m+1)^+$ | $^1$H-NMR ($CDCl_3$): δ = 0.1-0.3 (m,2H), 1.3-1.9 (m,13H), 1.9-2.5 (m,7H), 2.5-2.9 (m,7H), 3.1-3.3 (m,3H), 3.3-3.5(m,1H), 3.7-3.9 (m,2H), 4.9-5 (m,1H), 7.1 (m,1H), 7.3-7.5 (m,2H).<br>Found: C,56.42; H,6.92; N,7.36. $C_{27}H_{39}Cl_2N_3O_3S$. $H_2O$ requires C,56.44; H,7.19; N,7.31%. |

| Example No. | R | $R^3$ | $R^4$ | LRMS m/z = | Analytical data |
|---|---|---|---|---|---|
| 6[5] | 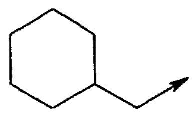 | Cl | Cl | 584.2 $(m+1)^+$ | $^1$H-NMR ($CDCl_3$): $\delta$ = 0.8-1.9 (m,18H), 1.9-2.3 (m,6H), 2.3-2.8 (m,8H), 3-3.6 (m,6H), 3.7-3.9 (m,2H), 7-7.1 (m,1H), 7.2-7.4 (m,2H). Found: C,58.62; H,7.54; N,6.90. $C_{29}H_{43}Cl_2N_3O_3S$. 0.5 $H_2O$ requires C,58.67; H,7.47; N,7.08%. |
| 7[6] | 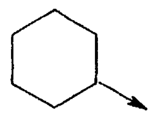 | F | F | 539.0 $(m+1)^+$ | $^1$H-NMR ($CDCl_3$): $\delta$ = 1-1.2 (m,4H), 1.3-1.5 (m,3H), 1.5-1.9 (m,10H), 1.9-2.3 (m,6H), 2.3-2.7 (m,5H), 2.8 (s,3H), 3.2-3.3 (m,3H), 3.4-3.5 (m,1H), 3.7-3.9 (m,2H), 4.4-4.6 (m,1H), 6.9-7.2 (m,3H). Found: C,61.23; H,7.75; N,7.56. $C_{28}H_{41}F_2N_3O_3S$. 0.5 $H_2O$ requires C,61.51; H,7.74; N,7.69%. |

Footnotes

1. See Preparation 9 for the preparation of the aldehyde starting material.
2. See WO-A-96/05193, Preparation 188, or WO-A-97/27185, Preparation 2, for the preparation of the aldehyde starting material.
3. See WO-A-96/05193, Preparation 138, for the preparation of the aldehyde starting material.
4. See Preparation 7 for the preparation of the aldehyde starting material.
5. See WO-A-96/05193, Preparation 139, for the preparation of the aldehyde starting material.
6. See Preparation 8 for the preparation of the aldehyde starting material.

EXAMPLE 8

(5S)-*1*-(Cyclopropylmethyl)-5-(3,4-dichlorophenyl)-5-(2-[3-[1-(ethylsulfonyl)-4-piperidinyl]-1-azetanyl]ethyl) tetrahydro-2(1*H*)-pyridinone

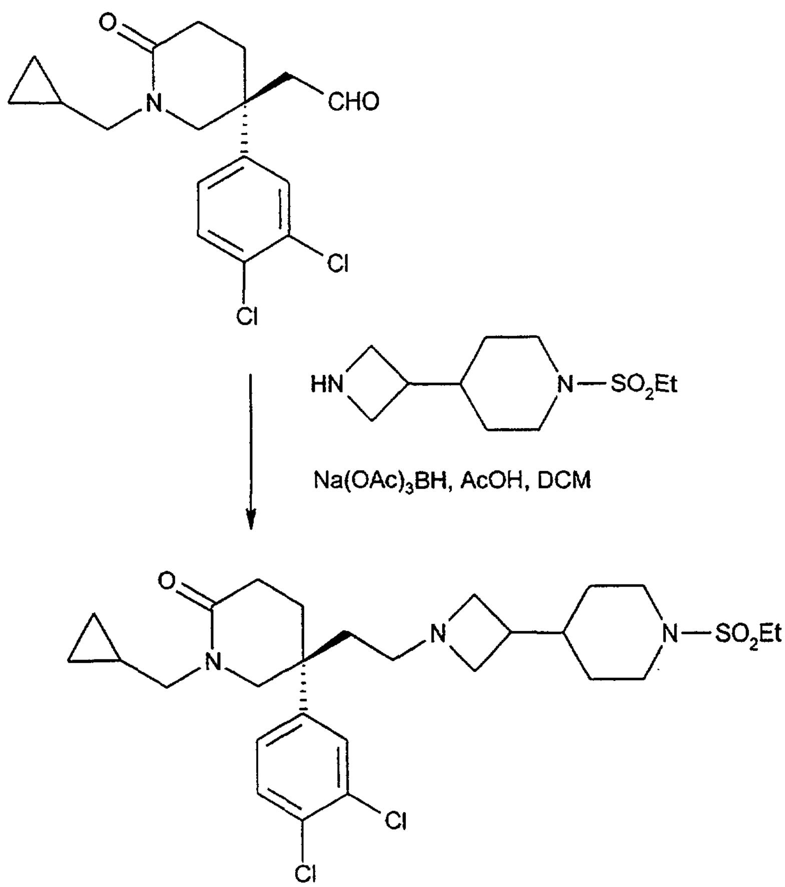


The title compound was prepared by a similar method to that of Example 1 using the appropriate aldehyde (see WO-A-96/05193, Example 123) and azetane (see Preparation 2) starting materials.
LRMS m/z = 558.3 $(m+1)^+$
Found: C,56.7; H,7.09; N,7.29. $C_{27}H_{39}Cl_2N_3O_3S$. $H_2O$ requires C,56.44; H,7.19; N,7.31%.
$^1$H-NMR ($CDCl_3$): δ=0.2-0.4 (m,2H), 0.5-0.7 (m,2H), 0.7-0.9 (m,1H), 1-1.25 (m,3H), 1.3 (m,3H), 1.4-1.9 (m,4H), 1.9-2.2 (m,6H), 2.2-2.5 (m,1H), 2.5-2.8 (m,4H), 2.8-3.0 (m,2H), 3.3-3.5 (m,2H), 3.6-3.8 (m,3H), 7.1-7.1(m,1H), 7.25-7.35 (m, 2H).

EXAMPLES 9-11

The compounds of the following tabulated Examples of the general formula:

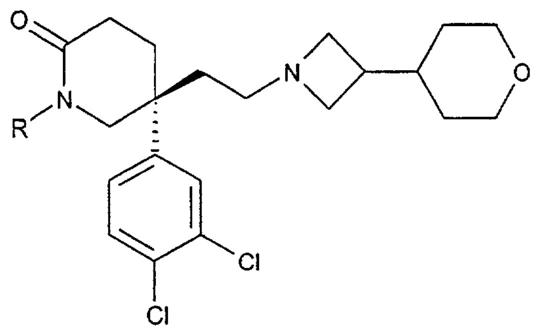

were prepared by a similar method to that of Example 1, except triethylamine was used instead of acetic acid, using the appropriate aldehyde and azetane (see Preparation 3) starting materials.

| Example No. | R | LRMS m/z = | Analytical data |
|---|---|---|---|
| 9 | 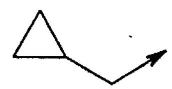 | 465.2 $(m+1)^+$ | $^1$H-NMR ($CDCl_3$): $\delta$ = 0.2-0.4 (m,2H), 0.5-0.7 (m,2H), 1-1.2 (m,3H), 1.4-1.7 (m,4H), 1.7-1.9 (m.1H), 1.9-2.3 (m,6H), 2.3-2.5 (m,1H), 2.6-2.8 (bs, 1H), 3.1-3.2 (m,1H), 3.2-3.6 (m,6H), 3.7-3.8 (m,1H), 3.8-4 (m,2H), 7.1-7.2 (m,1H), 7.3-7.5 (m,2H). Found: C,61.39; H,7.25; N,5.57. $C_{25}H_{34}Cl_2N_2O_2$. 0.5 $H_2O$ requires C,61.58; H,7.25; N,5.70%. |
| 10 | 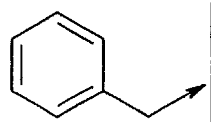 | 501.5 $(m+1)^+$ | $^1$H-NMR ($CDCl_3$): $\delta$ = 1-1.3 (m,2H), 1.4-1.6 (m,4H), 1.8-2 (m.1H), 1.9-2.3 (m,6H), 2.3-2.8 (bs, 2H), 3.0-3.1 (m,1H), 3.2-3.6 (m,6H), 3.7-3.8 (m,1H), 3.8-4 (m,2H), 7.1-7.2 (m,1H), 7.2-7.4 (m,7H). Found: C,64.87; H,7.03; N,5.30. $C_{28}H_{34}Cl_2N_2O_2$. $H_2O$ requires C,64.73; H,6.99; N,5.39%. |
| 11 | 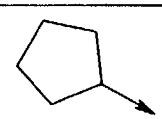 | 479.4 $(m+1)^+$ | $^1$H-NMR ($CDCl_3$): $\delta$ = 1-1.2 (m,2H), 1.3-1.9 (m,12H), 1.9-2.3 (7H), 2.3-2.5 (m,1H), 2.5-2.8 (m,2H), 3.1-3.5 (m,6H), 3.8-4 (m,2H), 4.8-5 (m,1H), 7-7.1 (m,1H), 7.3-7.5 (m,2H). Found: C,63.21; H,7.39; N,5.51. $C_{26}H_{36}Cl_2N_2O_2$. 0.5$H_2O$. 0.2 $CH_2Cl_2$ requires C,63.08; H,7.54; N,5.64%. |

The following Preparations illustrate the synthesis of certain intermediates used in the preparation of the

preceding Examples:-

PREPARATION 1

4-(3-Azetanyl)-1-(methylsulfonyl)piperidine[1]

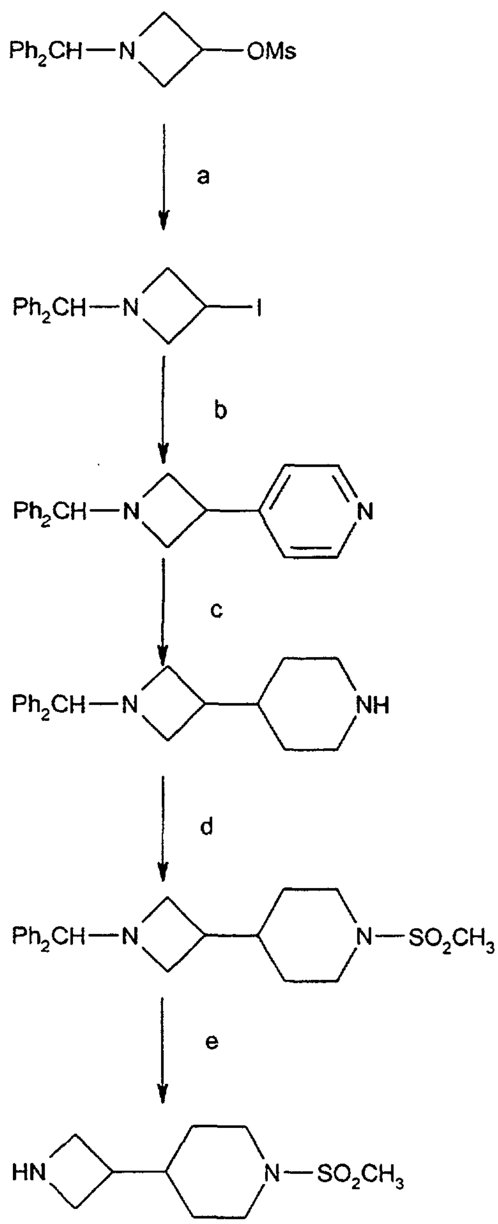


a) 1-Benzhydryl-3-iodoazetane

To a solution of 1-benzhydryl-3-methanesulfonyloxyazetane (see WO-A-96/05193) (60g, 0.189mol) in 1,2-dimethoxyethane (600ml) was added a solution of potassium iodide (60g) in water (300ml). The reaction was heated under reflux for 2.5 hours. After this time, the reaction was cooled to room temperature and then partitioned between ethyl acetate and dilute aqueous sodium carbonate solution. The organic portion was retained, dried over anhydrous sodium sulphate, then filtered. The solvent was removed from the filtrate by evaporation under reduced

pressure. The residue was chromatographed using silica gel eluting with diethyl ether. The required column fractions were combined, evaporated to dryness under reduced pressure, and the resultant residue crystallised from diisopropyl ether to provide the title compound (41g).
LRMS m/z = 282.2 $(m)^+$
$^1$H-NMR ($CDCl_3$): δ = 3.4-3.6 (m,2H), 3.8-4 (m,2H), 4.4-4.5 (m,1H), 4.7 (s,1H), 7.1-7.5 (m,10H).

b) 4-(1-Benzhydryl-3-azetanyl)pyridine

To a suspension of zinc dust (17g, 0.26mol) in tetrahydrofuran (40ml) under nitrogen was added 1,2-dibromoethane (2.1ml, 24mmol). The resultant mixture was heated to initiate the reaction, which became exothermic , and was heated under reflux for ca. 4 minutes. The reaction was allowed to cool to room temperature, then briefly heated to the reflux temperature again. After cooling to room temperature, trimethylsilyl chloride (2.5ml, 20mmol) was added resulting in a gas being evolved and an exothermic reaction. After stirring for a further hour, 1-benzhydryl-3-iodoazetane (70g, 0.20mol) in tetrahydrofuran (40ml) was added portionwise over 1-2 minutes. Upon complete addition, the reaction became strongly exothermic and the internal temperature was maintained between 28-32°C using a cold water bath. After the exotherm had subsided, the reaction was stirred at room temperature for a further 4 hours. After this time 4-chloropyridine (22.7g, 0.2mmol) was added, followed by dipalladium tribenzylideneacetone (2.8g, 3mmol) and tri-o-furyl phosphine (2.4g, 10mmol), and the reaction heated under reflux for 5 hours. The mixture was then cooled and stirring continued for a further 15 hours at room temperature. After this time the reaction was partitioned between dilute sodium carbonate solution and ethyl acetate. The aqueous layer was extracted a further three times with ethyl acetate and the combined organic portions dried over sodium sulphate. The organic portion was concentrated to dryness under reduced pressure and the residue purified by chromatography on silica gel eluting with a solvent gradient of ethyl acetate : pentane (80:20, by volume) changing to ethyl acetate. This provided the title compound as a yellow oil (14g).
LRMS m/z = 301.3 $(m+1)^+$
$^1$H-NMR ($CDCl_3$): δ=3.1-3.2 (m,2H), 3.5-3.7 (m,3H), 4.4 (s,1H), 7.1-7.5 (m,12H), 8.5 (m,2H).

c) 4-(1-Benzhydryl-3-azetanyl)piperidine

To a solution of 4-(1-benzhydryl-3-azetanyl)pyridine (29.7g, 99mmol) in methanol (300ml) was added aqueous hydrochloric acid (1.0M, 200ml), a further portion of methanol (100ml), and water (100ml). To this mixture was added platinum (IV) oxide (5.0g) and the mixture was hydrogenated overnight at 50°C and 414 kPa (60 p.s.i.). After this time the reaction was carefully filtered through Arbocel™ (caution - fire hazard) and the filtrate retained. The solvent was evaporated under reduced pressure to remove most of the water. A solution of sodium hydroxide (25g) in water (200ml) was added and the resultant mixture was extracted three times with diethyl ether. The ethereal portion was dried over sodium sulphate, filtered and concentrated under reduced pressure to produce a solid which was triturated with cold diisopropyl ether (50 ml) to provide the title compound as a solid (22.7g).
LRMS m/z = 307.2 $(m+1)^+$
$^1$H-NMR ($CDCl_3$): δ=0.8-1 (m,2H), 1.3-1.8 (m,4H), 2.1-2.3 (m,1H), 2.4-2.6 (m,2H), 2.6-2.8 (m,2H), 2.9-3.1 (m,2H), 3.2-3.4 (m,2H), 4.3 (s,1H), 7.1-7.4 (m,10H).

d) 4-(1-Benzhydryl-3-azetanyl)-1-(methylsulfonyl)piperidine

To a solution of 4-(1-benzhydryl-3-azetanyl)piperidine (22g, 72mmol) in dry dichloromethane (200ml) was added triethylamine (14ml, 0.1mol), and the mixture was cooled using an ice/acetone bath. To this mixture was added methanesulphonyl chloride (6.2ml, 80mmol), dropwise, over 10 minutes and reaction was allowed to stir for 15 hours. After this time a further portion of triethylamine (5ml) and methanesulphonyl chloride (0.7ml) were added and the reaction stirred for a further 2 hours. After this time the reaction was diluted with dichloromethane and washed with dilute sodium hydroxide solution. The organic portion was retained, dried over sodium sulphate and then filtered. The filtrate was concentrated under reduced pressure to provide a solid which was purified by column chromatography on silica gel eluting with a solvent gradient of ethyl acetate:pentane:dichloromethane (40:40:20, by volume) changing to ethyl acetate:pentane:dichloromethane (50:0:50 by volume) to give the title compound as a solid (15.1g).
LRMS m/z = 385.2 $(m+1)^+$
$^1$H-NMR ($CDCl_3$): δ=1.1-1.3 (m,2H), 1.4-1.6 (m,1H), 1.6-1.8 (m,2H), 2.1-2.3 (m,1H), 2.5-2.7 (m,2H), 2.7-2.8 (m,5H), 3.2-3.4 (m,2H), 3.7-3.8 (m,2H), 4.3 (s,1H), 7.1-7.4 (m,10H).

e) 4-(3-Azetanyly-1-(methylsulfonyl)piperidine

To a solution of 4-(1-benzhydryl-3-azetanyl)-1-(methylsulfonyl)piperidine (15.0g, 39mmol) in methanol

(400ml) was added aqueous hydrochloric acid (2.0M, 25ml). To this mixture was added palladium (II) hydroxide (20% w/w on carbon)(2.0g) and the mixture was hydrogenated for 5 hours at 60°C and 414 kPa (60 p.s.i.). After this time, the reaction was carefully filtered through Arbocel™ (caution - fire hazard) and the filtrate retained. The solvent was removed from the filtrate under reduced pressure to remove most of the water. The residue was then partitioned between dichloromethane and aqueous sodium hydroxide solution (1M). The dichloromethane portion was dried over sodium sulphate, filtered and concentrated under reduced pressure. The residue was triturated with cold diethyl ether (200 ml) to produce the title compound as a solid (6.8g).
LRMS m/z = 219.2 $(m+1)^+$
$^1$H-NMR ($CDCl_3$): δ = 1.1-1.3 (m,2H), 1.5-1.7 (m,1H), 1.7-1.8 (m,2H), 2-2.1 (s,1H), 2.4-2.5 (m,1H), 2.5-2.7 (m,2H), 2.8 (s,3H), 3.3-3.5 (m,2H), 3.6-3.7 (m,2H), 3.7-3.9 (m,2H).

Footnote

1. It should be noted that this compound may also be prepared by an analogous route to that described in this Preparation using a t-butyloxycarbonyl protecting group instead of the benzhydryl protecting group.

PREPARATION 2

4-(3-Azetanyl)-1-(ethylsulfonyl)piperidine

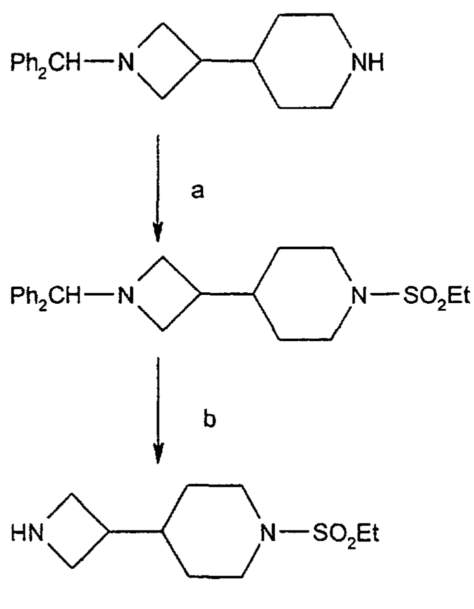


a) 4-(1-Benzhydryl-3-azetanyl)-1-(ethylsulfonyl)piperidine

A solution of 4-(1-benzhydryl-3-azetanyl)piperidine (see Preparation 1 (c)) (0.6g, 1.96mmol) and triethylamine (0.28ml, 1.96mmol) in dry dichloromethane (10ml) was cooled to -78°C under nitrogen. To this solution was added a solution of ethylsulphonyl chloride (0.186ml, 1.96mmol) in dry dichloromethane (10ml), dropwise, over 30 minutes and the reaction was allowed to warm slowly to room temperature over 3 hours. After this time the reaction was diluted with dichloromethane and washed with aqueous sodium carbonate solution. The aqueous layer was extracted with dichloromethane and the organic portions combined, dried over sodium sulphate, filtered and concentrated under reduced pressure. This provided the title compound as a white foam (0.72g).
LRMS m/z = 400 $(m+1)^+$
$^1$H-NMR ($CDCl_3$): δ = ($CDCl_3$): δ = 1.1-1.3 (m,2H), 1.3-1.4 (m,3H), 1.4-1.7 (m,3H), 2.1-2.3 (m,1H), 2.5-2.6 (m,1H), 2.7-2.8 (m,4H), 2.9 (m,2H), 3.2-3.4 (m,2H), 3.7-3.8 (m,2H), 4.3 (s,1H), 7.1-7.4 (m,10H).

b) 4-(3-Azetanyl)-1-(ethylsulfonyl)piperidine

This was prepared by a similar method to that used in Preparation 1(e) using the compound of Preparation 2(a) as the starting material.
LRMS m/z = 233.2 $(m+1)^+$
$^1$H-NMR ($CDCl_3$): $\delta$ = 1.1-1.3 (m,2H), 1.3-1.4 (m,3H), 1.4-1.7 (m,3H), 2.0-2.2 (m,1H), 2.5-2.6 (m,1H), 2.7-2.8 (m,4H), 2.9 (m,2H), 3.2-3.4 (m,2H), 3.6-3.8 (m,2H).

PREPARATION 3

3-(Tetrahydro-2H-pyran-4-yl)azetane hydrochloride

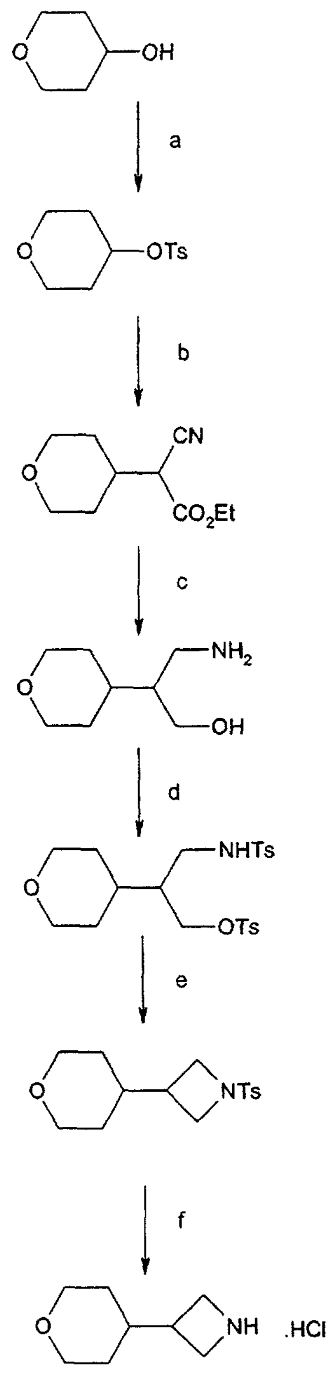


a) 4-(p-Tosyloxy)tetrahydro-2*H*-pyran

To an ice-cooled solution of tetrahydro-2*H*-pyran-4-ol (15g, 0.147mol) in dichloromethane (200ml) was added pyridine (36ml, 0.441 mol) followed by 4-methylbenzenesulfonyl chloride (56g, 0.294mol), portionwise. The resultant mixture was stirred at room temperature overnight. After this time, the mixture was partitioned between 2N aqueous

hydrochloric acid solution and dichloromethane. The aqueous portion was further extracted with dichloromethane and the organic fractions were combined and washed with brine. After drying over sodium sulphate and filtration, the solvent was removed under reduced pressure and the residue purified by column chromatography on silica gel using a gradient elution with diethyl ether:hexane (50:50, by volume) changing to diethyl ether:hexane (100:0, by volume). This produced the title compound as a colourless oil which solidified upon scratching the walls of the flask (30.3g).
LRMS m/z = 274.4 $(m+ NH_4)^+$
$^1$H-NMR ($CDCl_3$): δ = 1.6-1.9 (m,4H), 2.4 (s,3H), 3.4-3.5 (m,2H), 3.8-3.9 (m,2H), 4.6-4.7 (m,1H), 7.2-7.4 (m,2H), 7.7-7.8 (m,2H).

b) Ethyl (2-cyano-2-[tetrahydro-2*H*-pyran-4-yl])acetate

Freshly cut sodium (2.36g, 0.103mol) was added to ethanol (100ml) and stirred until all the sodium had dissolved. To this solution was added a solution of ethyl 2-cyanoacetate (11.5ml, 0.107mol) in ethanol (25ml), resulting in the formation of a white precipitate. The reaction was stirred under nitrogen and a solution of 4-(p-tosyloxy)tetrahydro-2H-pyran (25g, 0.0977mol) in ethanol (50ml) was added. The reaction was heated under reflux for 5 hours. After this time the solvent was removed under reduced pressure and the residue was partitioned between ethyl acetate and dilute aqueous citric acid solution. The organic portion was retained, dried over magnesium sulphate, filtered and concentrated under reduced pressure to give a red oil. The oil was purified by column chromatography on silica gel using a gradient elution with hexane : diethyl ether (2:1 changing to 1:1 changing to 1:2, by volume) to provide the title compound (7.9g).
LRMS m/z = 215.2 $(m+NH_4)^+$
$^1$H-NMR ($CDCl_3$): δ = 1.2-1.4 (m,3H), 1.5-1.8 (m,4H), 2.2-2.4 (m,1H), 3.3-3.5 (m,3H), 3.9-4.1 (m,2H), 4.2-4.4 (m,2H).

c) 3-Amino-2-(tetrahydro-2*H*-pyran-4-yl)-1-propanol

To an ice-cooled suspension of lithium aluminium hydride (2.94g, 77.4mmol) in dry diethyl ether (100ml) and dry tetrahydrofuran (100ml) was added a solution of ethyl (2-cyano-2-[tetrahydro-2*H*-pyran-4-yl])acetate (7.3g, 37mmol) in diethyl ether (50ml), dropwise, maintaining the internal temperature of the reaction at 5°C. The reaction was then stirred for 30 minutes at 5°C. After this time, tetrahydrofuran (100ml) was added, followed by a solution of water (3ml) in tetrahydrofuran (10ml), then 6N aqueous sodium hydroxide solution (3ml) and, finally, more water (8.8ml). The mixture was stirred for a further 20 minutes. The mixture was dried with potassium carbonate (150g) and filtered through a further 50g of potassium carbonate. The filter pad was washed with tetrahydrofuran and the combined organic portion concentrated under reduced pressure to provide the product as an oil.
LRMS m/z = 160.2 $(m+1)^+$
$^1$H-NMR ($CDCl_3$): δ = 0.2-0.6 (m,6H), 2-2.8 (bs,3H), 2.8-3 (m,1H), 3-3.2 (m,1H), 3.2-3.4 (m,2H), 3.6-3.8 (m,2H), 3.8-4 (m,2H).

d) 4-(3-[p-Tosyloxy]-1-[p-tosylamino]prop-2-yl)tetrahydro-2H-pyran

To a solution of 3-amino-2-(tetrahydro-2*H*-pyran-4-yl)-1-propanol (5.4g, 34mmol) in pyridine (30ml) under nitrogen was added p-tosyl chloride (16g, 84.9mmol) and 4-dimethylaminopyridine (catalytic amount). The reaction was allowed to stir at room temperature for 2 days. After this time the reaction was partitioned between ethyl acetate and dilute aqueous citric acid solution. The organic portion was retained and washed (x2) with further dilute aqueous citric acid solution, then with brine and finally dried over magnesium sulphate. After filtration, the solvent was removed under reduced pressure to give a crude solid which was purified by column chromatography on silica gel eluting with a solvent gradient of ethyl acetate:hexane (10:90 changing to 20:80 changing to 50:50, by volume) to provide the title compound as a white solid (7.01g).
LRMS m/z = 485.4 $(m+NH_4)^+$
$^1$H-NMR ($CDCl_3$): δ = 1.1-1.3 (m,2H), 1.3-1.4 (m,1H). 1.4.-1.5 (m, 1H), 1.5-1.7 (m,2H), 2.4-2.5 (s,s, 6H), 2.8-3 (m,2H), 3.1-3.3 (m,2H), 3.8-3.9 (m,2H), 3.9-4 (m,1H), 44.1 (m,1H), 4.7-4.8 (m,1H), 7.2-7.4 (m,4H), 7.6-7.8 (m,4H).

e) 1-[(4-Methylphenyl)sulfonyl]-3-(tetrahydro-2*H*-pyran-4-yl)azetane

To a solution of 4-(3-[p-tosyloxy]-1-[p-tosylamino]prop-2-yl)tetrahydro-2H-pyran (5.85g, 12.5mmol) in dry 1,2-dimethoxyethane (800ml) was added potassium-*tert*-butoxide (1.70g, 13.2mmol) and the mixture heated to 60°C for 20 minutes after which time a fine white solid was produced. The solvent was removed under reduced pressure and the residue partitioned between ethyl acetate and dilute aqueous citric acid solution. The organic portion was retained, dried over magnesium sulphate and chromatographed on silica gel eluting with ethyl acetate. Removal of the

solvent under reduced pressure provided the title compound (4.3g).
LRMS m/z = 313.5 $(m+NH_4)^+$
$^1$H-NMR ($CDCl_3$): δ = 0.9-1.5 (m,4H), 2.1-2.3 (m,1H), 2.4 (s,3H), 3.2-3.3 (m,2H), 3.4-3.5 (m,3H), 3.7-3.8 (2H), 3.8-3.9 (m,2H), 7.3-7.4 (m,2H), 7.7-7.8 (m,2H).

f) 3-(Tetrahydro-2*H*-pyran-4-yl)azetane hydrochloride

A solution of naphthalene (6.84g, 53.42mmol) in 1,2-dimethoxyethane (50ml) was cooled to -78°C using a dry-ice/acetone bath. To this solution, under nitrogen, was added freshly cut sodium (1.17g, 50.87mmol) and the reaction was stirred for 3 hours until a very dark green solution formed.
A solution of 1-[(4-methylphenyl)sulfonyl]-3-(tetrahydro-2*H*-pyran-4-yl)azetane (0.295g) in 1,2-dimethoxyethane (10ml) was cooled to -78°C using a dry-ice/acetone bath. To this was added the above, pre-formed lithium naphthalide solution until a persistent green colour was observed (after ca. 7ml of solution had been added). The reaction was then quenched with water (2ml) and allowed to warm to room temperature. Anhydrous potassium carbonate was added to dry the solution, the mixture was filtered and the solvent removed under reduced pressure. The residue was suspended in diethyl ether, filtered, concentrated under reduced pressure and purified by column chromatography on silica gel, eluting with a solvent gradient of methanol : dichloromethane : ammonia (5:95:0 changing to 5:95:0.5 changing to 10: 90:1, by volume). The appropriate column fractions were concentrated under reduced pressure, azeotroped with dichloromethane and the residue acidified with ethereal hydrogen chloride. The resultant solid was filtered and dried under reduced pressure at 80°C to provide the title compound (74mg).
LRMS 142.5 $(m+1)^+$
$^1$H-NMR ($CDCl_3$): δ = 1.1-1.3 (m,2H), 1.4-1.5 (m,2H), 1.8-1.9 (m,1H), 2.6-2.7 (m,1H), 3.2-3.4 (m,2H), 3.7-4.2 (m,6H), 9.3-9.8 (bs, 2H).

PREPARATION 4

(5S)-1-Cyclopentyl-5-(3,4-dichlorophenyl)-5-(1,3-dioxolan-2-ylmethyl)tetrahydro-2(1*H*)-pyridinone

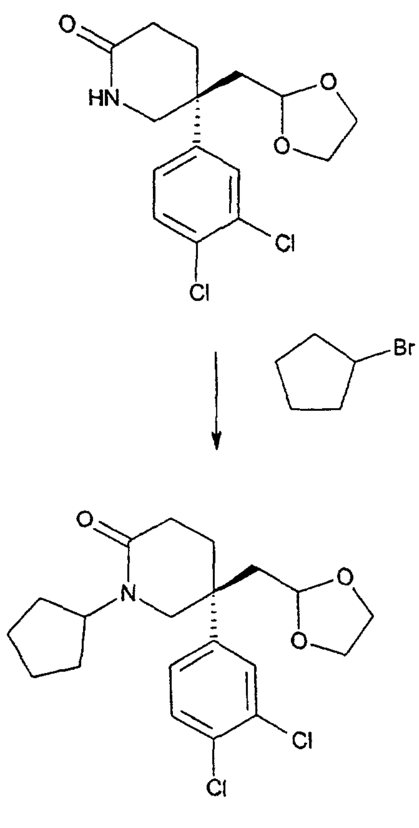

(5*S*)-5-(3,4-Dichlorophenyl)-5-(1,3-dioxolan-2-ylmethyl)tetrahydro-2(1*H*)-pyridinone (see WO-A-96/05193, Example 123) (10g, 30.3mmol) was suspended in 1,2-dimethoxyethane (250ml). To this mixture was added potassium *tert*-butoxide (3.4g, 30.3mmol) causing dissolution of the starting material. After 10 minutes, a solution of cyclopentyl bromide (3.25ml, 30.3mmol) in 1,2-dimethoxyethane (6.75ml) was added and the reaction was heated to 60°C. After 20 minutes at 60°C, a further quantity of potassium *tert*-butoxide (3.4g, 30.3mmol) was added followed by further cyclopentyl bromide (3.25ml, 30.3mmol) after 20 minutes. This further addition process was repeated 8 times. After this multiple addition process was completed, the reaction was partitioned between ethyl acetate and 2N aqueous sodium hydroxide solution. The organic layer was retained and the aqueous layer extracted with ethyl acetate. The organic portions were combined, washed with brine, dried over magnesium sulphate, filtered and concentrated under reduced pressure to give an orange/brown solid. This was purified by column chromatography on silica gel eluting with ethyl acetate to provide the title compound as a white, crystalline solid (9.3g).
LRMS m/z = 398.2 $(m)^+$
$^1$H-NMR ($CDCl_3$): $\delta$ = 1.5-1.9 (m,10H), 2-2.2 (m,4H), 2.4-2.5 (m,1H), 3.2-3.3 (m,1H), 3.5-3.6 (m,1H), 3.6-3.7 (m,1H), 3.8-3.9 (m,1H), 4.3-4.4 (m,1H), 4.8-5 (m,1H), 7-7.4 (m,3H).

PREPARATION 5

1-Cyclohexyl-5-(3,4-difluorophenyl)-5-(1,3-dioxolan-2-ylmethyl)tetrahydro-2(1*H*)-pyridinone

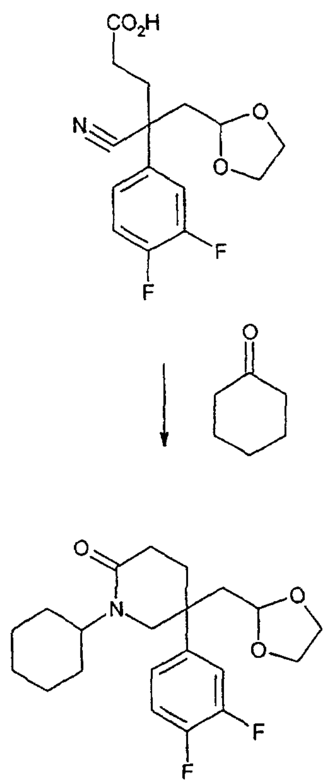

A solution of the nitrile (prepared in an analogous manner to that used to prepare 4(S)-4-cyano-4-(3,4-dichlorophenyl)-5-(1,3-dioxolan-2-yl)pentan-1-oic acid in WO-A-96/05193, Example 123) (9.8g, 31mmol) and cyclohexanone (16ml, 157mmol) in glacial acetic acid (50ml) was hydrogenated at 414 kPa (60 p.s.i.) and 50°C over platinum (IV) oxide (1.0g) for 15 hours. TLC analysis indicated that the reaction had proceeded 50% to completion. More cyclohexanone (16ml) and platinum oxide (1.0g) were added and hydrogenation continued for a further 15 hours. After this time, further cyclohexanone (16ml) and platinum oxide (1.0g) were added, and hydrogenation continued overnight. After this time the reaction was filtered through Arbocel™ (caution - fire hazard) and the filtrate concentrated under reduced pressure to yield a yellow oil. This residue was purified by column chromatography on silica gel using a gradiant elution with dichloromethane : methanol (100 : 0 changing to 98 : 2, by volume) to provide the title compound as a white solid (7.77g).

LRMS m/z = 380.2 $(m+1)^+$
$^1$H-NMR ($CDCl_3$): $\delta$ = 1-2 (m,11H), 2-2.3 (m,4H), 2.3-2.5 (m,1H), 3.3-3.4 (m,1H), 3.5-3.8 (m,3H), 3.8-4 (m,2H), 4.3-4.4 (m,1H), 4.4-4.6 (m,1H), 6.9-7.2 (m,3H).

PREPARATION 6

(5S)-1-Cyclohexyl-5-(3,4-difluorophenyl)-5-(1,3-dioxolan-2-ylmethyl)tetrahydro-2(1*H*)-pyridinone

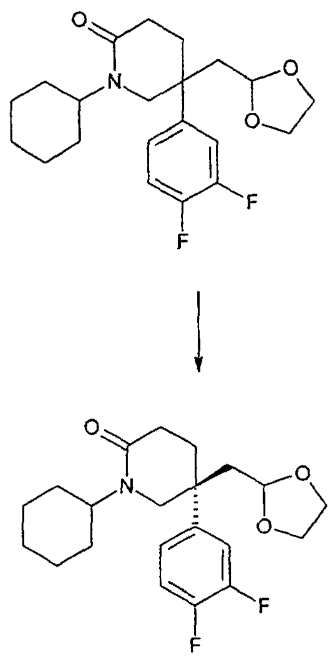

The racemic compound of Preparation 5 was resolved by chiral HPLC using a Chiralcel OD (trade mark) 250 x 20mm column and eluting with isopropanol:hexane (10:90, by volume) at a flow rate of 10ml/min, detecting peaks at 210nm. Peak 2 corresponded to the title compound that was then isolated.
$^1$H-NMR & LRMS were identical to those presented for the compound of Preparation 5.

PREPARATION 7

5(S)-1-Cyclopentyl-5-(3,4-dichlorophenyl-5-(formylmethyl)tetrahydro-2(1H)-pyridinone

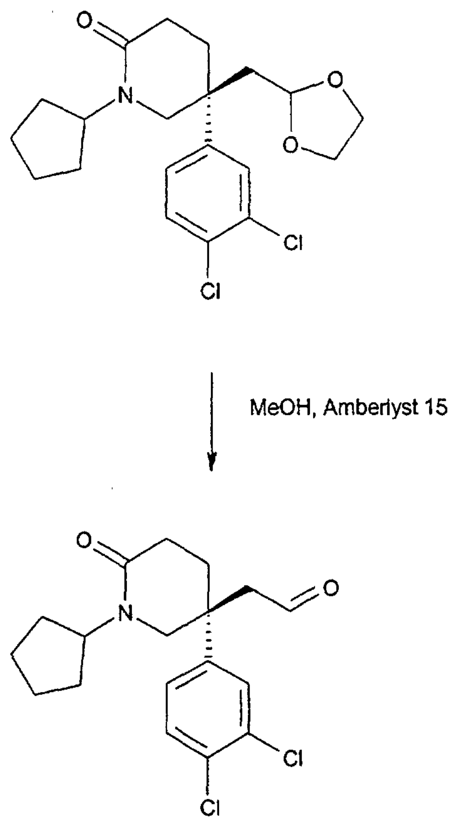


A mixture of the dioxolane (see Preparation 4) (9g, 22.6mmol), methanol (100ml) and Amberlyst 15 (trade mark) resin (9g) were stirred together for 24 hours. After this time the mixture was filtered and concentrated under reduced pressure. The residue was partitioned between ethyl acetate and water. The organic layer was retained and washed (x2) with water. After drying over sodium sulphate, the organic portion was filtered and concentrated under reduced pressure to an oil. This oil was dissolved in tetrahydrofuran (30ml) and aqueous hydrochloric acid solution (1 N, 30ml) was added. The mixture was stirred at room temperature overnight. After this time, the reaction was concentrated under reduced pressure to remove the tetrahydrofuran, and then extracted with ethyl acetate (x2). The organic portion was retained, dried over sodium sulphate, filtered and concentrated to provide an oil. This was purified by column chromatography on silica gel using a gradient elution with tetrahydrofuran : pentane, (50:50 changing to 66: 33 changing to 80:20, by volume) to provide the title compound (7.15g).

LRMS m/z = 354.2 $(m+1)^+$

$^1$H-NMR ($CDCl_3$): $\delta$ = 1.4-1.8 (m,9H), 2-2.5 (m,4H), 2.6-2.8 (m,1H), 2.8-3 (m,1H), 3.3-3.4 (m,1H), 3.5-3.6 (m,1H), 4.8-5 (m,1H), 7-7.4 (m,3H), 9.4 (s,1H).

PREPARATION 8

5(S)-1-Cyclohexyl-5-(3,4-difluorophenyl)-5-(formylmethyl)tetrahydro-2(1H)-pyridinone

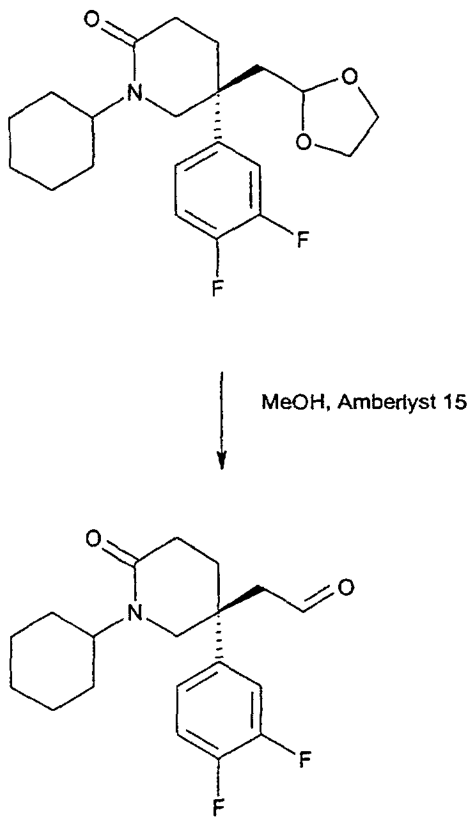


This compound was prepared by a similar method to that of Preparation 7 using the appropriate dioxolane starting material (see Preparation 6).
LRMS m/z = 337 $(m+1)^+$
$^1$H-NMR ($CDCl_3$): $\delta$ = 1-2 (m,10H), 2-2.5 (m,5H), 2.6-2.8 (m,1H), 2.8-3 (m,1H), 3.3-3.4 (m,1H), 3.6-3.8 (m,1H), 4.4-4.6 (m,1H), 7-7.3 (m,3H), 9.4 (s,1H).

PREPARATION 9

5(S)-1-Cyclopropylmethyl-5-(4-chloro-3-fluorophenyl)-5-(formylmethyl)tetrahydro-2(1H)-pyridinone

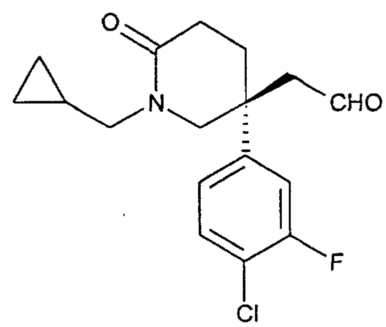

a) 4-Chloro-3-fluorophenylacetonitrile

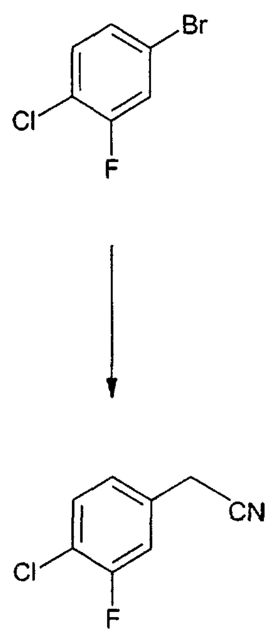

To a solution of 4-bromo-1-chloro-2-fluorobenzene (100g, 476mmol) in diisopropyl ether (500ml) at -78°C was added n-butyllithium (2.5M in hexane, 200ml, 500mmol), maintaining the temperature below -70°C. A white precipitate formed. After 30 minutes, zinc chloride (0.5M in THF, 1000ml, 500mmol ) was added, maintaining the temperature below -50°C . The resulting mixture was added to a solution of bromoacetonitrile (120g, 1000mmol), nickel(II) acetylacetonate (12.2g, 47.6mmol ) and tri-o-tolyphophine (14.5g, 47.6mmol ) in tetrahydrofuran (100ml), and the reaction heated under reflux for 2 hours. The reaction was concentrated under reduced pressure and partitioned between ethyl acetate and 2N aqueous sodium hydroxide solution. The organic layer was retained and the aqueous layer re-extracted with ethyl acetate . The organic portions were combined, washed with brine, dried over magnesium sulphate, filtered and concentrated under reduced pressure to give a brown oil. This was purified by column chromatography on silica gel eluting with a solvent gradient of diethyl ether:hexane (20:80, by volume) changing to diethyl ether:hexane (50:50, by volume) to provide the title compound as a red, crystalline solid (26.5g).

TLC $R_f$ = 0.25 (silica , diethyl ether:hexane, 1:1, by volume).
LRMS m/z = 169.0 $(m)^+$
$^1$H-NMR ($CDCl_3$): δ = 3.7 (s,2H), 7.05 (d,1H), 7.15 (d,1H) ,7.4 (dd, 1H).

b) 5(S)-1-Cyclopropylmethyl-5-(4-chloro-3-fluorophenyl)-5-(formylmethyl)tetrahydro-2(1H)-pyridinone

The title compound was prepared by an analogous method to that used to prepare 5(S)-1-cyclopropylmethyl-5-(3,4-dichlorophenyl)-5-formylmethyl-2-piperidone in WO-A-96/05193, Example 123, using the compound of Preparation 9(a) as the starting material.
LRMS m/z = 324.3 $(m+1)^+$.
$^1$H-NMR ($CDCl_3$): δ = 0.2-0.4 (m,2H), 0.5-0.7 (m,2H), 1-1.2 (m,1H), 2-2.3 (m,3H), 2.3-2.5 (m,1H), 2.6-3.1 (m,2H), 3.2-3.5 (m,2H), 3.6 (m,1H), 3.8-4 (m,1H), 7-7.4 (m,3H), 9.5 (m,1H).

PHARMACOLOGICAL DATA

The compounds of the present Examples and compounds described in the closest prior art, WO-A-96/05193, were tested for $NK_2$ receptor affinity and/or $NK_3$ receptor affinity and/or metabolic stability and the results are shown in Tables 1 and 2. These results show that the present compounds are, surprisingly, more potent $NK_2$ receptor antagonists and/or to have increased metabolic stability and/or to have improved selectivity as antagonists for the $NK_2$ receptor as opposed to the $NK_3$ receptor when compared to the compounds of WO-A-96/05193.
The affinity of the compounds of Tables 1 and 2 for the human $NK_2$ receptor was tested in vitro by testing their ability to compete with [$^3$H]-NKA for binding to membranes prepared from Chinese hamster ovary cells expressing the cloned human $NK_2$ receptor using the method set out on page 9.
The compounds of Tables 1 and 2 were tested for $NK_3$ receptor affinity, in vitro, by the method described on pages 9 and 10 by testing their ability to inhibit [$^3$H]-senktide binding to membranes from guinea pig cortex using the method of Guard, et al, Br.J.Pharmacol., 99, 767-773 (1990).
In Tables 1 and 2 the "$pK_i$" measurement is the negative logarithm of the molar affinity of the test compound for the receptor as determined in radioligand binding assays using standard protocols. A 1.0 log. unit difference in either the $NK_2$ or $NK_3$ receptor affinity figures corresponds to a 10-fold activity difference.
The metabolic stability of the compounds of Tables 1 and 2 was determined in vitro by the method described on page 10 according to the following experimental protocol.

Determination of metabolic stability *in vitro*

(a) Preparation of hepatic microsomes (HLM/37)

Transplant-quality human liver tissue was obtained from the International Institute for the Advancement of Medicine (Exton, PA). The donors ranged in age from 26 to 65 years old and included 3 males and 3 females. Hepatic microsomes were prepared from individual human livers by the process of differential centrifugation. Briefly, the liver tissue was homogenised in 50mM Tris HCI (pH 7.4) containing 250mM sucrose and then centrifuged at 9,000G for 20 minutes to remove the cell debris and nuclear fraction. The supernatant layer was removed and further centrifuged at 105,000G for 60 minutes to pellet the microsomal fraction. This pellet was washed with 100mM Tris HCI (pH 7.4) and centrifuged at 105,000G for 60 minutes to remove any contaminating haemoglobin. The final pellet was re-suspended in 100mM potassium phosphate (pH 7.4) and stored at -80°C prior to use. The cytochrome P450 content was determined using the method of Omura, T., Sato, R., J. Biol. Chem., 239, 2379-2385 (1964) and the protein concentration was determined using the method of Lowry *et al*., J. Biol. Chem., 193, 265-275 (1951), with bovine serum albumin as the protein standard.
The metabolic activities of the 6 major drug metabolising cytochrome P450 enzymes were determined for HLM/37 and compared to values obtained from a bank of individual human livers (n = 19).

| Cytochrome P450 | Lowest Activity | Highest Activity | Mean Activity | HLM/37 Activity |
|---|---|---|---|---|
| CYP1A2 | 3.6 | 37.9 | 7.3 | 6.1 |
| CYP2C9 | 536 | 7718 | 2100 | 2733 |
| CYP2C19 | 2.7 | 133.7 | 25.9 | 25.2 |
| CYP2D6 | 2.3 | 54.8 | 18.8 | 17.1 |

(continued)

| Cytochrome P450 | Lowest Activity | Highest Activity | Mean Activity | HLM/37 Activity |
|---|---|---|---|---|
| CYP2E1 | 0.2 | 2.4 | 1.2 | 1.3 |
| CYP3A4 | 69 | 6618 | 1348 | 1528 |

Based on this analysis HLM/37 appears to represent a "mean" human liver microsomal preparation.

(b) Incubations to determine disappearance half-life values

Each incubation (final volume 1.2ml) was comprised of microsomal protein (equivalent to 0.5μM cytochrome P450), 50mM Tris HCl (pH 7.4), 5mM $MgCl_2$ and 5μM $MnCl_2$. Reducing equivalents required for cytochrome P450 metabolism were provided by NADPH (1mM) that was regenerated in situ by an isocitric acid (5mM)/isocitric acid dehydrogenase (1 unit/ml) system (n.b. 1 unit/ml means that each ml of the incubation mixture contains 1 unit of isocitric acid dehydrogenase where 1 unit of this enzyme is defined as the amount of enzyme required to convert 1.0 micromole of isocitrate to alpha-ketoglutarate per min. at pH 7.4 and 37°C. The incubation mixture was pre-incubated at 37°C in the presence of the test compound (1μM) prior to addition of NADPH to initiate the reaction.

Aliquots (100μl) were removed from the incubation at 0, 3, 5, 10, 15, 20, 30, 45 and 60 minutes after the addition of NADPH. The reaction was terminated by immersion in ice-cold methanol (100μl). The resulting samples were centrifuged at 12,500 rpm for 5 minutes. After centrifugation 150μl aliquots were removed and 120μl of each of these analysed by HPLC on a Hypersil HS C18 5μ column (50x4.6mm) with a mobile phase of methanol:water (90: 10, by volume) containing 2mM ammonium acetate. Detection was by mass spectrometry using a Sciex API-100 single quadropole mass spectrometer monitoring the protonated molecular ion ($MH^+$) of the test compound. Analysis of the chromatograms was carried out using MacQuan 1.5.

The disappearance half-life for each incubation of test compound was obtained by plotting the natural log of the test compound peak area versus time. The slope of the line of best fit through the points yields the rate of metabolism (k). This was converted to a half-life using the following relationship:

$$\text{Half life } (t_{1/2}) = \frac{\ln 2}{k}$$

TABLE 1

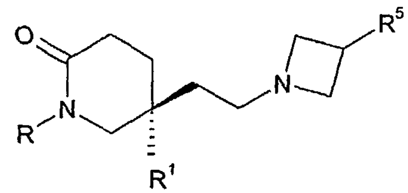

| Example No. | R | $R^1$ | $R^5$ | $NK_2$ receptor affinity ($pK_i$) | $NK_3$ receptor affinity ($pK_i$) | Metabolic stability ($t_{½}$)(min) | Close prior art in Table 2 |
|---|---|---|---|---|---|---|---|
| 1 | 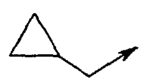 | 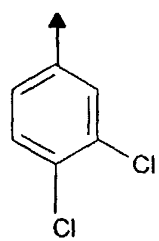 | 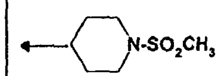 | 8.4 | - | 75 | Example 43 |
| 2 | 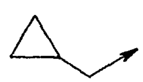 | 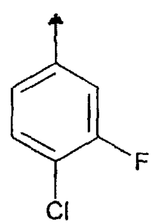 | 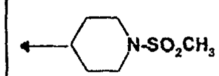 | 8.6 | - | >120 | Example 43 |

| | | | | | | | |
|---|---|---|---|---|---|---|---|
| 3 | 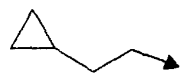 | 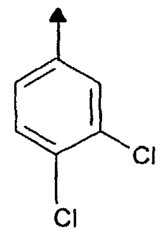 | 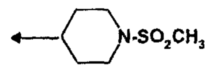 | - | - | 27 | Example 45 |
| 4 | 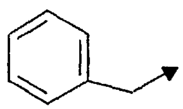 | 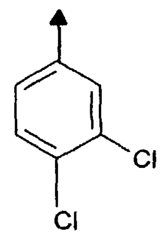 | 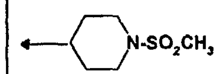 | 8.9 | - | - | (S) - enantiomer of racemate of Example 21 |
| 5 | 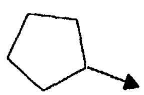 | 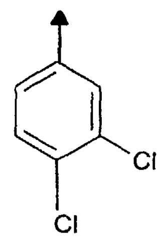 | 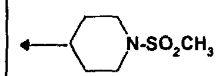 | 9.0 | 7.6 | - | (S) - enantiomer of racemate of either Example 71 or Example 90 |

| | | | | | | | |
|---|---|---|---|---|---|---|---|
| 6 | 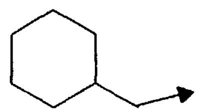 | 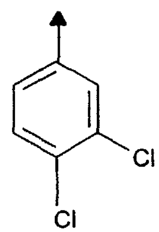 | 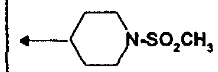 | 7.7 | 8.3 | - | (S) - enantiomer of racemate of Example 90 |
| 7 | 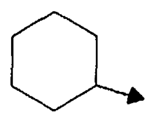 | 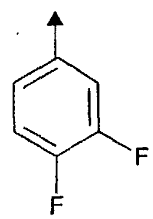 | 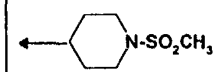 | 8.5 | - | - | (S) - enantiomer of racemate of Example 90 |
| 8 | 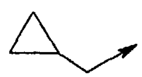 | 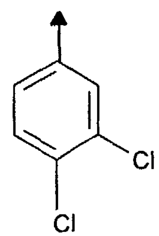 | 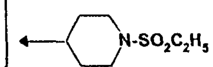 | 8.2 | - | - | Example 43 |

| | | | | | | | |
|---|---|---|---|---|---|---|---|
| 9 | 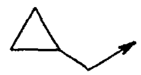 | 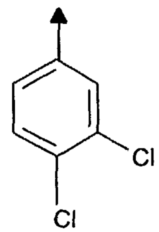 | 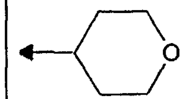 | 8.0 | - | - | Example 33 |
| 10 | 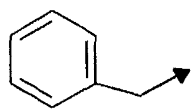 | 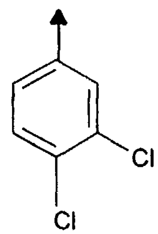 | 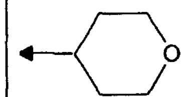 | 8.9 | - | - | Example 35 |
| 11 | 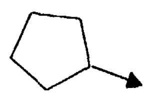 | 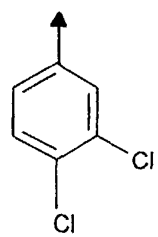 | 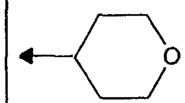 | 8.8 | 6.9 | - | (S) - enantiomer of racemate of Example 71 |

TABLE 2

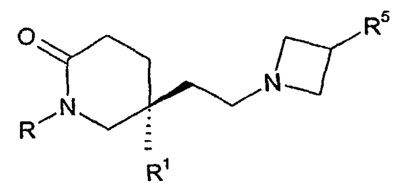

| WO-A-96/05193 Example No. | R | $R^1$ | $R^5$ | $NK_2$ receptor affinity ($pK_i$) | $NK_3$ receptor affinity ($pK_i$) | Metabolic stability ($t_{½}$)(min) |
|---|---|---|---|---|---|---|
| 43 | 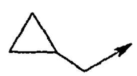 | 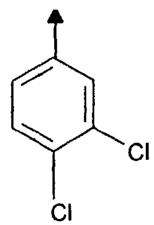 | 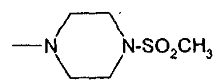 | 8.0 | - | 38 |
| 45 | 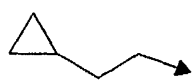 | 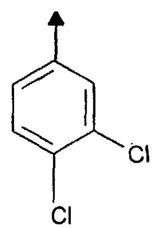 | 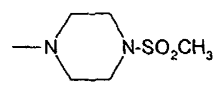 | - | - | 21 |

| | | | | | | |
|---|---|---|---|---|---|---|
| (S) - enantiomer of racemate of Example 21 | 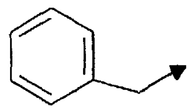 | 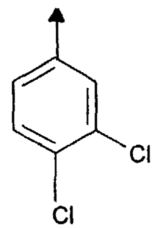 | 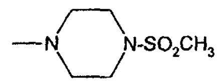 | 8.1 | - | - |
| (S) - enantiomer of racemate of Example 71 | 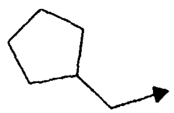 | 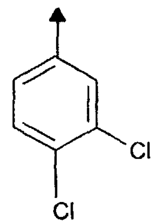 | 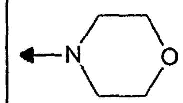 | 8.7 | 8.3 | - |
| (S) - Enantiomer of racemate of Example 90 | 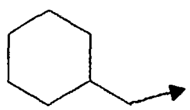 | 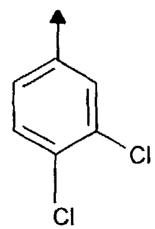 | 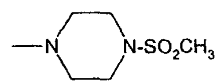 | 8.2 | 9.6 | - |

| | | | | | | |
|---|---|---|---|---|---|---|
| 33 | 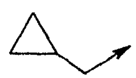 | 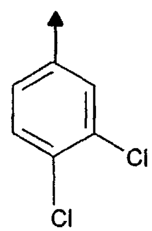 | 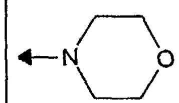 | 7.6 | - | - |
| 35 | 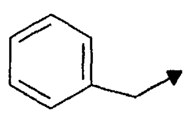 | 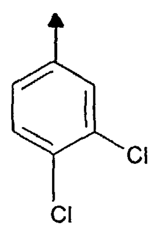 | 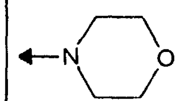 | 8.1 | - | - |

## Claims

1. A compound of the formula:-

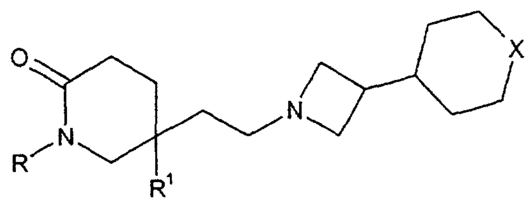

(I)

or a pharmaceutically acceptable acid addition salt or solvate thereof,
wherein

R is $C_3$-$C_7$ cycloalkyl, ($C_3$-$C_7$ cycloalkyl)$C_1$-$C_4$ alkylene or benzyl;
$R^1$ is phenyl optionally substituted by 1 or 2 substituents each independently selected from fluoro and chloro;
X is O or $NSO_2R^2$; and
$R^2$ is $C_1$-$C_4$ alkyl or halo($C_1$-$C_4$)alkyl.

2. A compound as claimed in claim 1 wherein the term "$C_3$-$C_7$ cycloalkyl" used in the definitions of R as $C_3$-$C_7$ cycloalkyl and ($C_3$-$C_7$ cycloalkyl)$C_1$-$C_4$ alkylene means cyclopropyl, cyclobutyl, cyclopentyl or cyclohexyl.

3. A compound as claimed in claim 1 or 2 wherein the term "$C_1$-$C_4$ alkylene" used in the definition of R as ($C_3$-$C_7$ cycloalkyl)$C_1$-$C_4$ alkylene means methylene or 1,2-ethylene.

4. A compound as claimed in claim 1 wherein the term "$C_1$-$C_4$ alkyl" used in the definitions of $R^2$ as $C_1$-$C_4$ alkyl and halo($C_1$-$C_4$)alkyl means methyl or ethyl.

5. A compound as claimed in claim 1 wherein R is ($C_3$-$C_7$ cycloalkyl)$C_1$-$C_4$ alkylene.

6. A compound as claimed in claim 1 wherein R is cyclopentyl, cyclohexyl, cyclopropylmethyl, 2-cyclopropylethyl, cyclohexylmethyl or benzyl.

7. A compound as claimed in claim 6 wherein R is cyclopropylmethyl.

8. A compound as claimed in any one of the preceding claims wherein $R^1$ is 3,4-difluorophenyl, 3,4-dichlorophenyl or 4-chloro-3-fluorophenyl.

9. A compound as claimed in claim 8 wherein $R^1$ is 3,4-dichlorophenyl.

10. A compound as claimed in any preceding claim wherein $R^2$ is $C_1$-$C_4$ alkyl.

11. A compound as claimed in any one of the preceding claims wherein X is $NSO_2$($C_1$-$C_4$ alkyl).

12. A compound as claimed in any one of claims 1 to 10 wherein X is O, $NSO_2CH_3$ or $NSO_2CH_2CH_3$.

13. A compound as claimed in claim 12 wherein X is $NSO_2CH_3$.

14. A compound as claimed in claim 1 having the (S)- stereochemistry at the 5-position of the piperidin-2-one ring, that is a compound of the formula (IA)

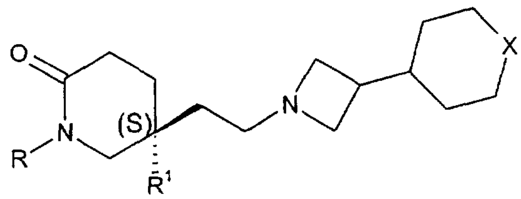

(IA)

wherein R, $R^1$ and X are as defined in any one of the preceding claims.

**15.** A compound as claimed in claim 1 which is 5(S)-1-(cyclopropylmethyl)-5-(3,4-dichlorophenyl)-5-(2-[3-(1-[methylsulfonyl]-4-piperidinyl)-1-azetanyl]ethyl)tetrahydro-2(1H)-pyridinone, or a pharmaceutically acceptable acid addition salt or solvate thereof.

**16.** A pharmaceutical composition comprising a compound of the formula (I), or a pharmaceutically acceptable acid addition salt or solvate thereof, as claimed in any one of the preceding claims, together with a pharmaceutically acceptable diluent or carrier.

**17.** A compound of the formula (I), or a pharmaceutically acceptable acid addition salt, solvate or composition thereof, as claimed in any one of claims 1 to 15 and 16, respectively, for use as a medicament.

**18.** The use of a compound of the formula (I), or of a pharmaceutically acceptable acid addition salt, solvate or composition thereof, as claimed in any one of claims 1 to 15 and 16, respectively, for the manufacture of a medicament for the treatment of a disease by producing an antagonist effect on a tachykinin acting at the human $NK_1$, $NK_2$ or $NK_3$ receptor, or any combination thereof.

**19.** Use as claimed in claim 18 where the disease is an inflammatory disease such as arthritis, psoriasis, asthma or inflammatory bowel disease, a central nervous system (CNS) disorder such as anxiety, depression, dementia or psychosis, a gastro-intestinal (GI) disorder such as functional bowel disease, irritable bowel syndrome, gastro-oesophageal reflux, faecal incontinence, colitis or Crohn's disease, an urogenital tract disorder such as incontinence, hyperreflexia or cystitis, a pulmonary disorder such as chronic obstructive airways disease, an allergy such as eczema, contact dermatitis or rhinitis, a hypersensitivity disorder such as to poison ivy, a peripheral neuropathy such as diabetic neuropathy, neuralgia, causalgia, painful neuropathy, a burn, herpetic neuralgia or post-herpetic neuralgia, cough or acute or chronic pain.

**20.** A compound of the formula:

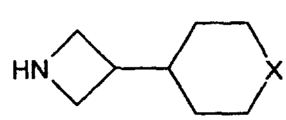

**(III)**

, or an acid addition salt thereof, wherein X is as defined in claim 1.

**21.** A compound of the formula:

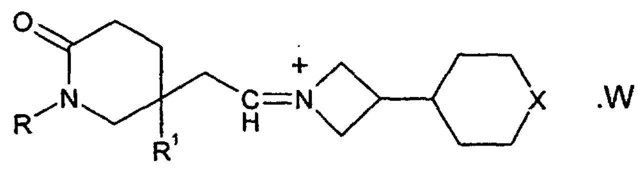

(IV)

where W is an anion derived from an acid and R, $R^1$ and X are as defined in claim 1.

**22.** A compound of the formula:

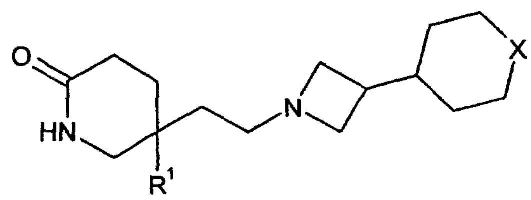

(V)

wherein $R^1$ and X are as defined in claim 1.

**23.** A process for the preparation of a compound of the formula (I) as claimed in claim 1 wherein R, $R^1$, $R^2$ and X are as defined in claim 1 which comprises:

(a) reductive animation reaction of a compound of the formula:

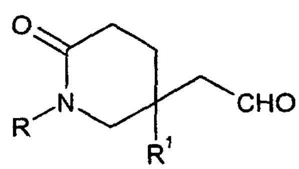

(II)

and a compound of the formula:

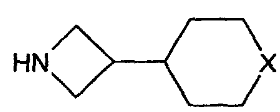

(III)

, or an acid addition salt thereof, wherein R, $R^1$ and X are as defined in this claim;

(b) alkylation of a N-deprotonated form of a compound of the formula:

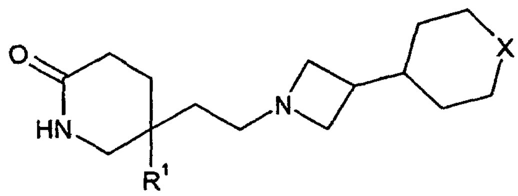

(V)

with a compound of the formula:

$$R\text{-}Y^1 \qquad (VI)$$

wherein $Y^1$ is a leaving group and R, $R^1$ and X are as defined in this claim; or

(c) reaction of a compound of the formula:

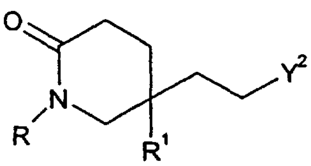

(VII)

wherein $Y^2$ is a leaving group and R and $R^1$ are as defined in this claim, with a compound of the formula:

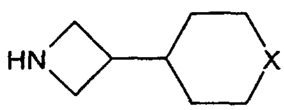

(III)

: any one of said processes (a) to (c) being followed by, optionally, conversion of a compound of the formula (I) to a pharmaceutically acceptable acid addition salt thereof.

**24.** A process as claimed in claim 23, part (a), wherein a reducing agent and an acid are present.

**25.** A process as claimed in claim 23, part (b), wherein $Y^1$ is halo, methanesulphonyloxy or para-toluenesulphonyloxy.

**26.** A process as claimed in claim 23, part (c), wherein $Y^2$ is chloro, bromo, iodo, methanesulphonyloxy, trifluoromethanesulphonyloxy or p-toluenesulphonyloxy.

## Patentansprüche

**1.** Verbindung der Formel:

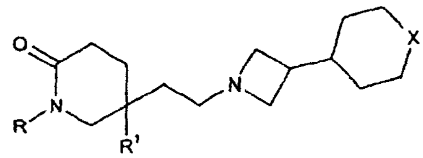

(I)

oder ein pharmazeutisch verträgliches Säureadditionssalz oder Solvat davon,

worin R $C_3$-$C_7$-Cycloalkyl, ($C_3$-$C_7$-Cycloalkyl)$C_1$-$C_4$-alkylen oder Benzyl darstellt;
$R^1$ Phenyl, gegebenenfalls substituiert mit 1 oder 2 Substituenten, jeweils unabhängig ausgewählt aus Fluor und Chlor, darstellt;
X O oder $NSO_2R^2$ darstellt; und
$R^2$ $C_1$-$C_4$-Alkyl oder Halogen($C_1$-$C_4$)alkyl darstellt.

**2.** Verbindung nach Anspruch 1, worin der in den Definitionen von R als $C_3$-$C_7$-Cycloalkyl und ($C_3$-$C_7$-Cycloalkyl) $C_1$-$C_4$-alkylen verwendete Begriff "$C_3$-$C_7$-Cycloalkyl" Cyclopropyl, Cyclobutyl, Cyclopentyl oder Cyclohexyl bedeutet.

**3.** Verbindung nach Anspruch 1 oder 2, worin der in der Definition von R als ($C_3$-$C_7$-Cycloalkyl)$C_1$-$C_4$-alkylen verwendete Begriff "$C_1$-$C_4$-Alkylen" Methylen oder 1,2-Ethylen bedeutet.

**4.** Verbindung nach Anspruch 1, worin der in den Definitionen von $R^2$ als $C_1$-$C_4$-Alkyl und Halogen($C_1$-$C_4$)alkyl verwendete Begriff "$C_1$-$C_4$-Alkyl" Methyl oder Ethyl bedeutet.

**5.** Verbindung nach Anspruch 1, worin R ($C_3$-$C_7$-Cycloalkyl)$C_1$-$C_4$-alkylen darstellt.

**6.** Verbindung nach Anspruch 1, worin R Cyclopentyl, Cyclohexyl, Cyclopropylmethyl, 2-Cyclopropylethyl, Cyclohexylmethyl oder Benzyl darstellt.

**7.** Verbindung nach Anspruch 6, worin R Cyclopropylmethyl darstellt.

**8.** Verbindung nach einem der vorangehenden Ansprüche, worin $R^1$ 3,4-Difluorphenyl, 3,4-Dichlorphenyl oder 4-Chlor-3-fluorphenyl darstellt.

**9.** Verbindung nach Anspruch 8, worin $R^1$ 3,4-Dichlorphenyl darstellt.

**10.** Verbindung nach einem vorangehenden Anspruch, worin $R^2$ $C_1$-$C_4$-Alkyl darstellt.

**11.** Verbindung nach einem der vorangehenden Ansprüche, worin X $NSO_2$($C_1$-$C_4$-Alkyl) darstellt.

**12.** Verbindung nach einem der Ansprüche 1 bis 10, worin X O, $NSO_2CH_3$ oder $NSO_2CH_2CH_3$ darstellt.

**13.** Verbindung nach Anspruch 12, worin X $NSO_2CH_3$ darstellt.

**14.** Verbindung nach Anspruch 1 mit der (S)-Stereochemie an der 5-Position des Piperidin-2-on-Rings nämlich eine Verbindung der Formel (IA)

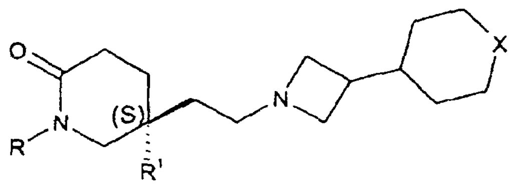

(IA)

worin R, $R^1$ und X wie in einem der vorangehenden Ansprüche definiert sind.

**15.** Verbindung nach Anspruch 1, nämlich 5(S)-1-(Cyclopropylmethyl)-5-(3,4-dichlorphenyl)-5-(2-[3-(1-[methylsulfonyl]-4-piperidinyl)-1-azetanyl]ethyl)tetrahydro-2(1H)-pyridinon oder ein pharmazeutisch verträgliches Säureadditionssalz oder Solvat davon.

**16.** Pharmazeutische Zusammensetzung, umfassend eine Verbindung der Formel (I) oder ein pharmazeutisch verträgliches Säureadditionssalz oder Solvat davon nach einem der vorangehenden Ansprüche zusammen mit einem pharmazeutisch verträglichen Verdünnungsmittel oder Träger.

**17.** Verbindung der Formel (I) oder ein pharmazeutisch verträgliches Säureadditionssalz, Solvat oder eine Zusammensetzung davon nach einem der Ansprüche 1 bis 15 bzw. 16 zur Verwendung als ein Arzneimittel.

**18.** Verwendung einer Verbindung der Formel (I) oder eines pharmazeutisch verträglichen Säureadditionssalzes, Solvats oder einer Zusammensetzung davon nach einem der Ansprüche 1 bis 15 bzw. 16 zur Herstellung eines Arzneimittels für die Behandlung einer Erkrankung durch Erzeugen einer Antagonistenwirkung auf ein Tachykinin, das an dem Human-$NK_1$-, -$NK_2$- oder -$NK_3$-Rezeptor oder einer beliebigen Kombination davon wirkt.

**19.** Verwendung nach Anspruch 18, wobei die Erkrankung eine entzündliche Erkrankung, wie Arthritis, Psoriasis, Asthma oder entzündliche Darmerkrankung, eine Störung des zentralen Nervensystems (ZNS), wie Angstzustand, Depression, Demenz oder Psychose, eine Störung des Gastrointestinaltrakts (GI), wie funktionelle Darmkrankheit, reizbares Darmsyndrom, gastrooesophagealer Reflux, Stuhlinkontinenz, Colitis oder Crohnsche Krankheit, eine Störung des Urogenitaltraktes, wie Inkontinenz, Hyperreflexie oder Zystitis, eine Luftwegsstörung, wie chronische obstruktive Luftwegserkrankungen, eine Allergie, wie Ekzem, Kontaktdermatitis oder Schnupfen, eine Hypersensibilitätsstörung, wie auf Giftsumach, eine periphere Neuropathie, wie diabetische Neuropathie, Neuralgie, Kausalgie, schmerzvolle Neuropathie, eine Verbrennung, herpetische Neuralgie oder post-herpetische Neuralgie, Husten oder akuter oder chronischer Schmerz darstellt.

**20.** Verbindung der Formel:

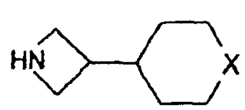

(III)

oder ein Säureadditionssalz davon, worin X wie in Anspruch 1 definiert ist.

**21.** Verbindung der Formel :

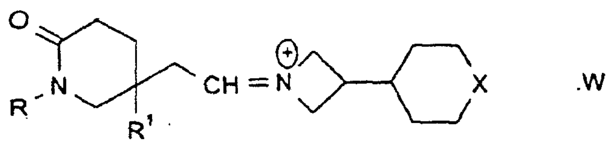

(IV)

worin W ein von einer Säure abgeleitetes Anion darstellt und R, $R^1$ und X wie in Anspruch 1 definiert sind.

**22.** Verbindung der Formel:

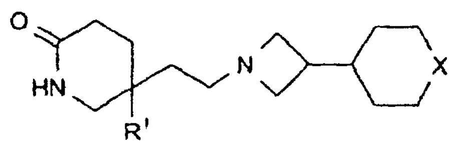

(V)

worin $R^1$ und X wie in Anspruch 1 definiert sind.

**23.** Verfahren zur Herstellung einer Verbindung der Formel (I) wie in Anspruch 1 definiert, worin R, $R^1$, $R^2$ und X wie in Anspruch 1 definiert sind, das umfasst:

(a) reduktive Aminierungsreaktion einer Verbindung der Formel:

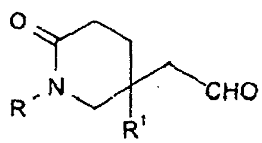

(II)

und einer Verbindung der Formel:

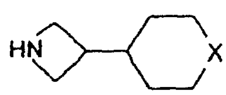

(III)

oder eines Säureadditionssalzes davon, worin R, $R^1$ und X, wie in diesem Anspruch definiert sind;
(b) Alkylierung einer Verbindung der Formel:

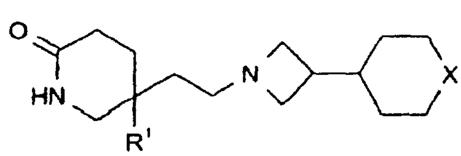

(V)

die von N-Schutzgruppen befreit wurde, mit einer Verbindung der Formel:

$$R\text{-}Y^1 \quad (VI)$$

worin $Y^1$ eine Abgangsgruppe darstellt und R, $R^1$ und X wie in diesem Anspruch definiert sind; oder (c) eine Umsetzung einer Verbindung der Formel:

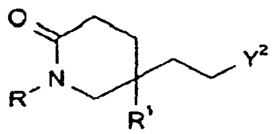

(VII)

worin $Y^2$ eine Abgangsgruppe darstellt und R und $R^1$ wie in diesem Anspruch definiert sind, mit einer Verbindung der Formel

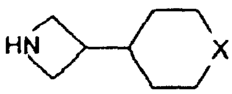

(III)

wobei jedem dieser Verfahren (a) bis (c) gegebenenfalls Umwandlung einer Verbindung der Formel (I) in ein pharmazeutisch verträgliches Säureadditionssalz davon folgt.

**24.** Verfahren nach Anspruch 23, Teil (a), wobei ein Reduktionsmittel und eine Säure vorliegen.

**25.** Verfahren nach Anspruch 23, Teil (b), wobei $Y^1$ Halogen, Methansulfonyloxy oder para-Toluolsulfonyloxy darstellt.

**26.** Verfahren nach Anspruch 23, Teil (c), wobei $Y^2$ Chlor, Brom, Jod, Methansulfonyloxy, Trifluormethansulfonyloxy oder p-Toluolsulfonyloxy darstellt.

## Revendications

**1.** Composé de formule :

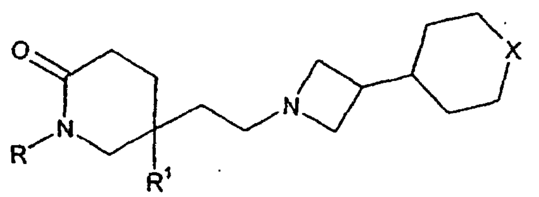

(I)

ou un de ses sels d'addition d'acide ou produits de solvatation pharmaceutiquement acceptables, formule dans laquelle R représente un groupe cycloalkyle en $C_3$ à $C_7$, (cycloalkyle en $C_3$ à $C_7$) (alkylène en $C_1$ à $C_4$) ou benzyle ;

$R^1$ représente un groupe phényle facultativement substitué avec un ou deux substituants choisis chacun indépendamment entre des substituants fluoro et chloro,
X représente O ou un groupe $NSO_2R^2$ ; et
$R^2$ représente un groupe alkyle en $C_1$ à $C_4$ ou halogénalkyle en $C_1$ à $C_4$.

**2.** Composé suivant la revendication 1, dans lequel l'expression "cycloalkyle en $C_3$ à $C_7$" utilisée dans les définitions de R en tant que groupes cycloalkyle en $C_3$ à $C_7$ et (cycloalkyle en $C_3$ à $C_7$)(alkylène en $C_1$ à $C_4$) désigne un groupe cyclopropyle, cyclobutyle, cyclopentyle ou cyclohexyle.

**3.** Composé suivant la revendication 1 ou 2, dans lequel l'expression "alkylène en $C_1$ à $C_4$" utilisée dans la définition de R en tant que groupe (cycloalkyle en $C_3$ à $C_7$) (alkylène en $C_1$ à $C_4$) désigne un groupe méthylène ou 1,2-éthylène.

**4.** Composé suivant la revendication 1, dans lequel l'expression "alkyle en $C_1$ à $C_4$" utilisée dans les définitions de $R^2$ en tant que groupes alkyle en $C_1$ à $C_4$ et halogénalkyle en $C_1$ à $C_4$ désigne un groupe méthyle ou éthyle.

**5.** Composé suivant la revendication 1, dans lequel R représente un groupe (cycloalkyle en $C_3$ à $C_7$) (alkylène en $C_1$ à $C_4$).

**6.** Composé suivant la revendication 1, dans lequel R représente un groupe cyclopentyle, cyclohexyle, cyclopropylméthyle, 2-cyclopropyléthyle, cyclohexylméthyle ou benzyle.

**7.** Composé suivant la revendication 6, dans lequel R représente un groupe cyclopropylméthyle.

**8.** Composé suivant l'une quelconque des revendications précédentes, dans lequel $R^1$ représente un groupe 3,4-difluorophényle, 3,4-dichlorophényle ou 4-chloro-3-fluorophényle.

**9.** Composé suivant la revendication 8, dans lequel $R^1$ représente un groupe 3,4-dichlorophényle.

**10.** Composé suivant l'une quelconque des revendications précédentes, dans lequel $R^2$ représente un groupe alkyle en $C_1$ à $C_4$.

**11.** Composé suivant l'une quelconque des revendications précédentes, dans lequel X représente un groupe $NSO_2$ (alkyle en $C_1$ à $C_4$).

**12.** Composé suivant l'une quelconque des revendications 1 à 10, dans lequel X représente O, un groupe $NSO_2CH_3$ ou $NSO_2CH_2CH_3$.

**13.** Composé suivant la revendication 12, dans lequel X représente un groupe $NSO_2CH_3$.

**14.** Composé suivant la revendication 1, ayant la stéréochimie (S) en position 5 du noyau pipéridine-2-one, c'est-à-dire composé de formule (IA)

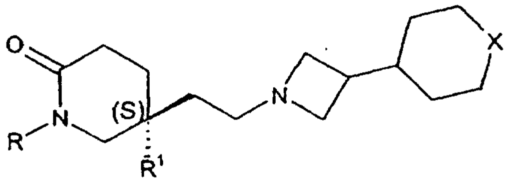

(IA)

dans laquelle R, $R^1$ et X répondent aux définitions suivant l'une quelconque des revendications précédentes.

**15.** Composé suivant la revendication 1, qui est la 5(S)-1-(cyclopropylméthyl)-5-(3,4-dichlorophényl)-5-(2-[3-(1-[méthylsulfonyl]-4-pipéridinyl)-1-azétanyl]éthyl)-tétrahydro-2(1H)-pyridinone ou un de ses sels d'addition d'acide ou produits de solvatation pharmaceutiquement acceptables.

**16.** Composition pharmaceutique comprenant un composé de formule (I), ou un de ses sels d'addition d'acide ou produits de solvatation pharmaceutiquement acceptables, suivant l'une quelconque des revendications précédentes, conjointement avec un diluant ou support pharmaceutiquement acceptable.

**17.** Composé de formule (I) ou un de ses sels d'addition d'acide, ou produits de solvatation pharmaceutiquement acceptables ou bien une composition le contenant, suivant l'une quelconque des revendications 1 à 15 et 16, respectivement, destiné à être utilisé comme médicament.

**18.** Utilisation d'un composé de formule (I), ou d'un de ses sels d'addition d'acide ou produits de solvatation pharmaceutiquement acceptables ou bien d'une composition le contenant, suivant l'une quelconque des revendications 1 à 15 et 16, respectivement, pour la production d'un médicament destiné au traitement d'une maladie en produisant un effet antagoniste sur une tachykinine agissant au niveau du récepteur $NK_1$, $NK_2$ ou $NK_3$ humain ou de n'importe laquelle de leurs associations.

**19.** Utilisation suivant la revendication 18, dans laquelle la maladie est une maladie inflammatoire telle que l'arthrite, le psoriasis, l'asthme ou une maladie intestinale inflammatoire, un trouble du système nerveux central (SNC) tel que l'anxiété, la dépression, la démence ou la psychose, un trouble gastro-intestinal (GI) tel qu'une maladie intestinale fonctionnelle, le syndrome du côlon irritable, le reflux gastro-oesosophagien, l'incontinence fécale, la colite ou la maladie de Crohn, un trouble du tractus urogénital tel que l'incontinence, l'hyperréflexie ou la cystite, un trouble pulmonaire tel qu'une maladie obstructrice chronique des voies aériennes, une allergie telle que l'eczéma, la dermatite de contact ou la rhinite, un trouble d'hypersensibilité tel que l'empoisonnement par le sumac vénéneux, une neuropathie périphérique telle qu'une neuropathie diabétique, une névralgie, une causalgie, une neuropathie douloureuse, une brûlure, une névralgie herpétique ou une névralgie post-herpétique, la toux, ou une douleur aiguë ou chronique.

**20.** Composé de formule :

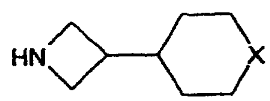

**(III)**

ou un de ses sels d'addition d'acide, formule dans laquelle X répond à la définition suivant la revendication 1.

**21.** Composé de formule :

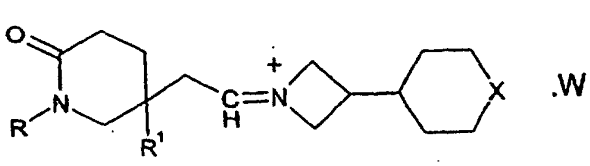

**(IV)**

dans laquelle W représente un anion dérivé d'un acide et R, $R^1$ et X répondent aux définitions suivant la revendication 1.

**22.** Composé de formule :

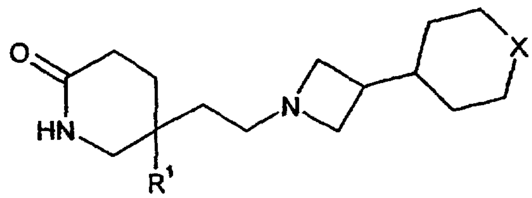

(V)

dans laquelle $R^1$ et X répondent aux définitions suivant la revendication 1.

**23.** Procédé pour la préparation d'un composé de formule (I) suivant la revendication 1, dans lequel R, $R^1$, $R^2$ et X répondent aux définitions suivant la revendication 1, qui comprend

(a) une réaction d'amination réductrice d'un composé de formule :

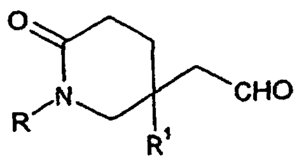

(II)

et d'un composé de formule :

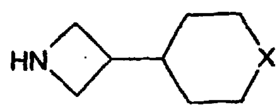

(III)

ou d'un de ses sels d'addition d'acide, formules dans lesquelles R, $R^1$ et X répondent aux définitions figurant dans cette revendication ;
(b) l'alkylation d'une forme N-déprotonée d'un composé de formule :

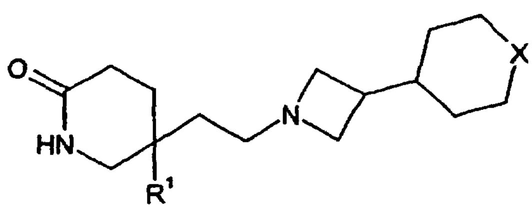

(V)

avec un composé de formule :

$$R\text{-}Y^1 \quad (VI)$$

formules dans lesquelles $Y^1$ représente un groupe partant et R, $R^1$ et X répondent aux définitions figurant dans cette revendication ; ou

(c) la réaction d'un composé de formule :

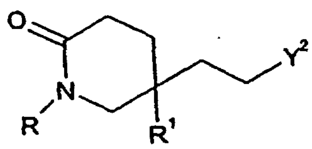

(VII)

dans laquelle $Y^2$ représente un groupe partant et R et $R^1$ répondent aux définitions figurant dans cette revendication, avec un composé de formule :

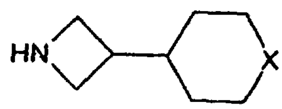

(III)

l'un quelconque desdits procédés (a) à (c) étant suivi, facultativement, par la conversion d'un composé de formule (I) en un de ses sels d'addition d'acide pharmaceutiquement acceptables.

**24.** Procédé suivant la revendication 23, partie (a), dans lequel un agent réducteur et un acide sont présents.

**25.** Procédé suivant la revendication 23, partie (b), dans lequel $Y^1$ représente un groupe halogéno, méthanesulfonyloxy ou paratoluènesulfonyloxy.

**26.** Procédé suivant la revendication 23, partie (c), dans lequel $Y^2$ représente un groupe chloro, bromo, iodo, méthanesulfonyloxy, trifluorométhane-sulfonyloxy ou p-toluènesulfonyloxy.